(19) Europäisches Patentamt
European Patent Office
Office européen des brevets

(11) EP 1 385 982 B1

(12) **EUROPÄISCHE PATENTSCHRIFT**

(45) Veröffentlichungstag und Bekanntmachung des Hinweises auf die Patenterteilung:
**22.10.2008 Patentblatt 2008/43**

(21) Anmeldenummer: **02714122.5**

(22) Anmeldetag: **28.01.2002**

(51) Int Cl.:
***C12Q 1/00*** *(2006.01)* ***C07K 7/06*** *(2006.01)*
***G01N 33/542*** *(2006.01)*

(86) Internationale Anmeldenummer:
**PCT/EP2002/000845**

(87) Internationale Veröffentlichungsnummer:
**WO 2002/059352 (01.08.2002 Gazette 2002/31)**

(54) **VERFAHREN UND MITTEL ZUR DETEKTION ENZYMATISCHER SPALT- UND VERKNÜPFUNGSREAKTIONEN**

METHODS AND MEANS FOR DETECTING ENZYMATIC CLEAVAGE AND LINKAGE REACTIONS

PROCEDES ET MOYENS DE DETECTION DE REACTIONS DE CRAQUAGE ET DE RETICULATION A CATALYSE ENZYMATIQUE

(84) Benannte Vertragsstaaten:
**AT BE CH CY DE DK ES FI FR GB GR IE IT LI LU MC NL PT SE TR**

(30) Priorität: **26.01.2001 EP 01101869**

(43) Veröffentlichungstag der Anmeldung:
**04.02.2004 Patentblatt 2004/06**

(73) Patentinhaber: **Evotec AG**
**22525 Hamburg (DE)**

(72) Erfinder:
- **LOPEZ-CALLE, Eloisa**
  **67059 Ludwigshafen (DE)**
- **FRIES, Joachim**
  **4802 Strengelbach (DE)**
- **JUNGMANN, Joern**
  **22559 Hamburg (DE)**

(74) Vertreter: **Meyers, Hans-Wilhelm et al**
**Deichmannhaus am Dom**
**50667 Köln (DE)**

(56) Entgegenhaltungen:
**WO-A-00/52199** **WO-A-00/72016**
**FR-A- 2 791 141** **US-A1- 2001 046 668**

- **SINGH K K ET AL: "FLUORESCENCE POLARIZATION FOR MONITORING RIBOZYME REACTIONS IN REAL TIME" BIOTECHNIQUES, EATON PUBLISHING, NATICK, US, Bd. 29, Nr. 2, August 2000 (2000-08), Seiten 344-351, XP001093639 ISSN: 0736-6205**
- **LEVINE LEANNA M ET AL: "Measurement of specific protease activity utilizing fluorescence polarization." ANALYTICAL BIOCHEMISTRY, Bd. 247, Nr. 1, 1997, Seiten 83-88, XP002228468 ISSN: 0003-2697 in der Anmeldung erwähnt**
- **ROBLES JORDI ET AL: "Synthesis and enzymatic stability of phosphodiester-linked peptide-oligonucleotide hybrids" BIOCONJUGATE CHEMISTRY, AMERICAN CHEMICAL SOCIETY, WASHINGTON, US, Bd. 8, Nr. 6, November 1997 (1997-11), Seiten 785-788, XP002175521 ISSN: 1043-1802**
- **BERGMANN F ET AL: "Solid phase synthesis of directly linked peptide-oligodeoxynucleotide hybrids using standard synthesis protocols" TETRAHEDRON LETTERS, ELSEVIER SCIENCE PUBLISHERS, AMSTERDAM, NL, Bd. 36, Nr. 11, 13. März 1995 (1995-03-13), Seiten 1839-1842, XP004028500 ISSN: 0040-4039**
- **ROBLES J ET AL: "Towards Nucleopeptides Containing Any Trifunctional Amino Acid" TETRAHEDRON, ELSEVIER SCIENCE PUBLISHERS, AMSTERDAM, NL, Bd. 55, Nr. 46, 12. November 1999 (1999-11-12), Seiten 13251-13264, XP004180925 ISSN: 0040-4020**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 385 982 B1

## Beschreibung

Die Erfindung betrifft Verfahren zur Detektion enzymkatalysierter Spalt- und Verknüpfungsreaktionen sowie Mittel, insbesondere Kits und Substrate, zur Durchführung dieser Verfahren.

Enzymatische Spaltreaktionen wie beispielsweise insbesondere proteolytische Reaktionen sind in der Biochemie weit verbreitet und spielen in der biologischen Funktion von Organismen eine entscheidende Rolle. Deshalb ist es eine wichtige Aufgabe, in geeigneten Testverfahren, sogenannten Assays, diese Reaktionen zu verfolgen. Die Funktionsfähigkeit eines Assays ist entscheidend geprägt von seiner Zuverlässigkeit und Reproduzierbarkeit. Vor allem an Assaysysteme, wie sie im Hochdurchsatzscreening nach neuen pharmakologisch aktiven Substanzen benutzt werden, sind sehr hohe Maßstäbe in dieser Hinsicht zu stellen, da die Zeit, die zur Durchführung des Assays und insbesondere zur Detektion eines Messpunktes verbleibt, extrem kurz ist. Die derzeit bekannten Assaysysteme und Substrate für insbesondere proteolytische Reaktionen genügen in vielerlei Hinsicht nicht diesen Ansprüchen.

Bekannt sind beispielsweise Assays für unspezifische proteolytische Enzyme auf Basis bakterieller Luciferase als Substrat (Njus et al., Analytical Biochemistry 61, 280 - 287, 1974), welche naturgemäß versagen, wenn Enzyme mit einer genau definierten Substratspezifität untersucht werden sollen.

Im Stand der Technik sind zudem Assaysysteme mit den dazugehörigen Substraten beschrieben, die auf fluorogenen oder chromogenen Prinzipien unter Verwendung eines Farbstoffs zur Kopplung an das Substrat beruhen. Das fluorogene Prinzip basiert auf der Synthese eines Amides aus einer primären oder sekundären Amin-Einheit eines Farbstoffes mit der Carboxylgruppe einer Aminosäure. Die elektronenziehende Eigenschaft der Carboxylgruppe bewirkt, dass es zu einer Verringerung der Elekronendichte im Farbstoffsystem kommt (dies gilt im übrigen auch für das chromogene Prinzip) und somit das chromophore System gestört wird, d. h. der Farbstoff verliert seine Farbstoffeigenschaften. An die angekoppelte Aminosäure kann nun ein Peptid beliebiger Sequenz synthetisiert werden. Entscheidend in jedem Fall ist, dass die Spaltstelle für die proteolytische Reaktion die Amid-Bindung zwischen Farbstoff und erster Aminosäure darstellt. Durch die Spaltung dieser Bindung wird die Störung des elektronischen Systems des Farbstoffes aufgehoben, er erhält seine Farbstoffeigenschaften zurück (chromogenes System) und ist - im Falle eines Fluoreszenzfarbstoffes - wieder in der Lage, nach geeigneter Anregung Lichtquanten zu emittieren (fluorogenes System). Ein derartiges System ist beispielsweise in den US - Patenten 4,557,862 und 4,640,893 offenbart, welche eine Klasse von Rhodaminderivaten beschreiben, die als Bisamidsubstitutionsprodukte nicht fluoreszent sind, während die Monoamidsubstitutionsprodukte ein hohes Maß an Fluoreszenz aufweisen. Spaltung einer einzigen spezifischen Amidbindung durch spezifische Proteasen führt somit das nichtfluoreszente Derivat in ein fluoreszentes Derivat über, welches als Ausleseparameter für die proteolytische Reaktion dient. Anwendungen dieser Techniken für Serin-Proteasen wurden beispielsweise beschrieben durch Leytus et al. (Biochem. J. 209, 299 - 307, 1983; Biochem. J. 215, 253 - 260, 1983). Die Techniken wurden ebenfalls für Cystein-Protease-Assays - z. B. zur Detektion der für apoptotische Prozesse relevanten Enzymklasse der Caspasen - eingesetzt, siehe z. B. Hug et al. (Biochemistry 38, 13906 - 13911, 1999), Weber et al. (WO 99/18856), Xiang et al. (Proc. Natl. Acad. Sci. USA, 93, 14559 - 14563, 1996) und Talanian et al. (The Journal of Biological Chemistry, 272, 9677 - 9682, 1997).

Die Nachteile dieser Techniken liegen in der sehr eingeschränkten Auswahl an Farbstoffen, die in der beschriebenen Art mit Aminosäuren verbunden werden können und die dadurch reversibel ihre Farbstoffeigenschaften verlieren. Die wenigen bekannten Beispiele stellen Rhodamin 110 und 7-Aminomethylcumarin dar. Die Synthese dieser Moleküle ist teilweise sehr schwierig. Zudem handelt es sich bei der Amidbindung zwischen Farbstoff und Aminosäure nicht um eine peptidische Bindung, wie sie bei realen biologischen Substraten einer Proteinase vorliegen. Dies führt zu einer dramatischen Verschlechterung der molekularen Erkennung durch das Enzym. Deshalb muß hier in einem Konzentrationsbereich gearbeitet werden, wie er für Einzelmolekültechniken z. B. auf konfokaler Basis nicht mehr tolerierbar ist. Als Ausleseparameter dient die Fluoreszenzintensität, welche schwierig zu normieren ist, so dass hieraus nur sehr problematisch die Parameter der zu untersuchenden proteolytischen Reaktion abgeleitet werden können. Ein hohes Maß an Hintergrundfluoreszenz, wie es bei diesen Substraten auftritt, führt ebenfalls zu einer Erniedrigung der Brauchbarkeit eines solchen Systems. Aufgrund des gestörten elektronischen Systems des Farbstoffs vor erfolgter enzymatischer Reaktion findet keine Emission statt und somit kann nicht kontrolliert werden, wie viel Substrat vorgelegt worden ist.

Des Weiteren sind im Stand der Technik Assays unter Verwendung von Substraten, die mit zwei Farbstoffmolekülen markiert sind, beschrieben. Proteolytische Assays dieser Art enthalten eine Peptidsequenz, die bevorzugt eine spezifische Spaltsequenz trägt und mit zwei Farbstoffmarkern an den beiden Enden der Peptidkette markiert ist. Durch die proteolytische Reaktion wird die Peptidkette in zwei (bei einer Spaltstelle) oder mehrere Bruchstücke zerlegt und die beiden Farbstoffmarker räumlich voneinander getrennt. Im Falle einer heterogenen Markierung handelt es sich um ein Farbstoffpaar, das zu Fluoreszenz-Resonanz-Energie-Transfer (FRET) befähigt ist. Durch die proteolytische Reaktion wird dieser FRET-Effekt zerstört und beide spektral getrennten Farbstoffe besitzen ihre jeweiligen charakteristischen Fluoreszenzeigenschaften, die vorher in der ungespaltenen Peptidkette in Wechselwirkung miteinander standen. FRET-Messungen wurden beispielsweise an fluorogenen Substraten für Interleukin-1$\beta$ Converting Enzyme (ICE) durchgeführt (Pennington et al., Peptide Research, 7, 72 - 76, 1994). Der FRET-Effekt beruht auf der räumlichen Orientierung der beiden beteiligten Partner zueinander, so dass Fluktuationen im Abstand, wie sie in einer nicht räumlich fixierten Peptid-Kette gegeben sind, auch zu Schwankungen im Signal führen, was eine sichere Detektion stört. Ein weiterer Nachteil ist, dass die Spaltstelle zwischen den beiden Farbstoffen des FRET-Paares liegen muss. Durch diese räumliche Nähe kann es zur Interferenz mit der enzymatischen Bindung kommen. Bei einer homogenen Markierung handelt es sich um zwei identische Farbstoffmolküle, z. B. Tetramethylrhodamin wie von Packard et al. beschrieben (J. Phys. Chem. B 102, 1820 - 1827, 1998; J. Phys. Chem. B 102, 752 - 758, 1998; Biophysical Chemistry, 67, 167 - 176, 1997). Tetramethylrhodamin zeigt in wässriger Umgebung die Tendenz, hydrophobe Wechselwirkungen auszubilden, die zu einer Dimerisierung des Farbstoffes und zu einem Verlust seiner Fluoreszenzeigenschaften führen. Dies bedeutet, dass die beiden Farbstoffmoleküle gemeinsam an einem Peptid intramolekular dimerisieren und erst nach Spaltung der Peptidkette diese intramolekulare Dimerisierung aufgehoben wird. Eine intermolekulare Dimerisierung nach erfolgter Spaltung ist zwar nach wie vor möglich, aber aufgrund der Konzentrationsverhältnisse, in denen gearbeitet wird, nur wenig relevant. Allerdings ist auch die Dimerisierung der Farbstoffe im ungespaltenen Zustand nicht vollständig, so dass ein Hintergrundsignal detektierbar bleibt. Weiterhin können naturgemäß nur Farbstoffe mit hydrophoben Eigenschaften - denn nur diese zeigen die Dimerisierungsneigung - genutzt werden, was zu erheblichen Problemen beim Aufbau eines Assays führt, der in biologisch kompatibler und somit wässriger Umgebung eingesetzt werden muss.

Im Stand der Technik sind ferner proteolytische Assays auf Fluoreszenzanisotropie-Basis beschrieben. Diese arbeiten nach dem Prinzip, dass kleine mit Fluoreszenzfarbstoffen markierte Bruchstücke nach der proteolytischen Reaktion eine höhere Mobilität und damit eine schnellere Rotation als ein Gesamtmolekül vor der Reaktion besitzen. Zum einen kann es sich bei dem Gesamtmolekül um ein großes, statistisch in einem hohen Grade markiertes Protein handeln, das keine spezifische Spaltsequenz gegenüber einer bestimmten Protease besitzt. Das Protein wird in Bruchstücke zerlegt, die zu einem gewissen Teil Fluoreszenzmarker tragen. Ein derartiger auf der Fluoreszenzpolarisationsbestimmung als Detektionsmethode der Wahl aufbauender Assay und geeignete Substrate sind beispielsweise durch Maeda (Analytical Chemistry 92, 222 - 227, 1979) beschrieben.

Zum anderen kann es sich um ein Peptid mit einer bevorzugt spezifischen Spaltsequenz handeln, das auf der einen Seite einen Farbstoff und auf der anderen ein Biotin-Molekül trägt. Über eine Biotin-(Strept-)Avidin-Wechselwirkung entsteht ein sehr großer Komplex mit hoher Anisotropie. Die spezifische Spaltsequenz, die in das Peptid eingebaut wurde, wird durch die proteolytische Reaktion gespalten und es entsteht ein kleines farbstofftragendes Peptid-Bruchstück (siehe z. B. Bolger et al., BioTechniques 17, 586 - 589, 1994; Levine et al., Analytical Biochemistry 247, 83 - 88, 1997). Allerdings kann je nach eingesetzter Protease auch das (Strept-)Avidin-Protein angegriffen werden, so dass keine Spezifität gegeben ist, da auch eine proteolytische Reaktion, die nicht für die eingebaute Spaltsequenz spezifisch ist, durch Spaltung des (Strept)Avidins eine entsprechende Reaktion vortäuschen kann. Selbst durch die nachträgliche Zugabe von (Strept-)Avidin kann dieser Nachteil nicht ausgeglichen werden, so dass dieses System unter Umständen unbrauchbar sein kann. Ohnehin ist bei nachträglicher Zugabe von (Strept-)Avidin nur eine Endpunktbestimmung duchführbar und eine Beobachtung des Reaktionsverlaufes nicht möglich. Ein derartiges biotinyliertes Substrat, welches erst nach erfolgter enzymatischer Reaktion mit (Strept-)Avidin behandelt wird, um eine Detektion zu ermöglichen, ist nicht für kinetische Experimente geeignet, da nach Zugabe von (Strept-)Avidin häufig auch die Reaktion gestoppt wird. Unterschiedliche Reaktionszeiten wären somit nur durch entsprechend viele Reaktionsansätze zu realisieren. Aber selbst der genannte Stoppeffekt ist nicht kalkulierbar, wie eigene Experimente mit Caspase-3 zeigen, in denen ein enzymatischer Umsatz des mit (Strept-)Avidin versetzten Caspase-3-Substrates wider Erwarten auftrat. Zusammenfassend läßt sich also sagen, dass derartige Assaysysteme den Anforderungen hinsichtlich Verlässlichkeit der erzielten Resultate sowie Robustheit bei der Durchführung nicht gerecht werden.

Ein Verfahren zur Detektion enzymkatalysierter Spaltreaktionen und dessen Verwendung ist in WO 00/72016 A offenbart. Dabei wird ein erstes fluoreszentes Reagenz eingesetzt, das nicht-kovalent an ein nach der Spaltung zugesetztes Modul hoher Molmasse bindet.

Ferner offenbaren Singh et al. (K. K. Singh, T. Rücker, A. Hanne, R. Parwaresch und G. Krupp, BioTechniques 29:344-351, August 2000) eine Anwendung der Fluoreszenzpolarisation von fluoreszenten RNA-Substraten zur Überwachung von makromolekularen Reaktionen.

Aufgabe der vorliegenden Erfindung ist es daher, universell einsetzbare Verfahren zur Detektion von enzymatischen Spalt- und Verknüpfungsreaktionen sowie korrespondierende Mittel bereitzustellen, welche die oben genannten Nachteile nicht aufweisen und insbesondere zum Einsatz in Hochdurchsatzverfahren zur Suche nach pharmakologisch aktiven Modulatoren der enzymatischen Reaktion(en) geeignet sind.

Diese Aufgaben werden gelöst durch Assayverfahren gemäß der unabhängigen Ansprüche.

In einem ersten Aspekt betrifft die Erfindung somit ein Verfahren zur Detektion enzymkatalysierter Spaltreaktionen mit folgenden Schritten:

a) Bereitstellen einer modularen chemischen Verbindung enthaltend das folgende Sequenzmotiv

Z - X - Y oder Y - X - Z

wobei Z ein Modul wählbarer Molmasse umfasst, welches bezüglich der enzymatischen Spaltreaktion inert ist,
X ein Modul mit n Spaltstellen $S_1$ bis $S_n$ umfasst, welches durch die enzymkatalysierte Spaltreaktion in mindestens zwei, $X_1$ bzw.
$X_{n+1}$ enthaltende Spaltprodukte definierter Molmasse spaltbar ist, und n eine ganze Zahl ≥ 1 darstellt,
Y ein fluoreszentes Reportermodul umfasst, und wobei die Bestandteile der Verbindung Z - X - Y oder Y- X - Z kovalent miteinander verbunden sind
als Substrat für das/die die Spaltreaktion(en) katalysierende(n) Enzyme;

b) Inkubieren der Verbindung mit dem/den die Spaltreaktion(en) katalysierenden Enzym(en) unter Entstehung mindestens zweier Spaltprodukte, welche enthalten

$$Z - X_1 \text{ und } X_{n+1} - Y$$

oder

$$Y - X_1 \text{ und } X_{n+1} - Z$$

wobei die Molmasse des Z - $X_1$ bzw. $X_{n+1}$ - Z enthaltenden Spaltproduktes mindestens fünfzig Prozent der Gesamtmolmasse des Substrates beträgt;

c) Detektieren der enzymatischen Aktivität(en) durch Bestimmung des das Reportermodul Y enthaltenden Spaltproduktes mittels eines molmassensensitiven Verfahrens, welches auf bevorzugt konfokaler Fluoreszenzkorrelationsspektroskopie, Fluoreszenzpolarisationsbestimmung und/oder Fluoreszenzanisotropiebestimmung beruht.

Von besonderer Bedeutung für das erfindungsgemäße Verfahren ist zum einen der Einsatz eines bezüglich der enzymatischen Spaltreaktion inerten Modules Z. So könnten bei Verwendung eines nichtinerten Modules Z beispielsweise Fälle auftreten, in denen Reaktionsprodukte entstehen, die sich hinsichtlich ihrer Molmasse nur geringfügig unterscheiden und durch molmassensensitive Verfahren nicht oder nur mit großer Ungenauigkeit auslesbar wären. Würde das Modul Z durch die enzymatische Aktivität an mehreren Stellen gespalten, so würde eine Vielzahl von Reaktionsprodukten unterschiedlicher Molmasse entstehen mit der Konsequenz eines "verschmierten" Detektionssignales. Andererseits ist es ferner von besonderer Bedeutung, einen hinreichend hohen Molmassenunterschied zwischen dem Substrat und dem das Reportermodul Y enthaltenden Spaltprodukt zu gewährleisten, um eine hohe Detektionsgenauigkeit zu ermöglichen. Insbesondere in Hochdurchsatzscreeningverfahren (HTS) nach pharmakologisch wirksamen Modulatoren der Enzymreaktion ist die Detektionszeit oftmals auf nur wenige Sekunden pro Modulator begrenzt, so dass eine überdurchschnittlich hohe Detektionsgenauigkeit unabdingbar ist. Diese Anforderungen werden erfindungsgemäß dadurch gewährleistet, dass die Molmasse des Moduls Z derart zu wählen ist, dass nach erfolgter enzymatischer Reaktion die Molmasse des Z-$X_1$ bzw. $X_{n+1}$-Z enthaltenden Spaltproduktes mindestens fünfzig Prozent der Gesamtmolmasse des generischen Substrates beträgt, und dass die Bestandteile der einzelnen Substrate der enzymatischen Verknüpfungsreaktion(en) kovalent miteinander verbunden sind.

Die Bestandteile des Substrates sind kovalent miteinander verbunden. Die besondere Bedeutung der kovalenten Verknüpfung der Substratbestandteile zeigt sich in eindrucksvoller Weise bei einem Vergleich mit dem (Strept-)Avidin-Biotin-System des Standes der Technik. Beispielsweise könnten in einer Substanzbibliothek, die im Hochdurchsatzscreening getestet werden soll, Substanzen existieren, welche die Bindung von (Strept-)Avidin an Biotin beeinflussen. Bei einem Screening nach Enzyminhibitoren bzw. Enzymaktivatoren würden diese Substanzen als falsch-negative bzw. -positive Hits aufscheinen. Eine Eliminierung falschpositiver Hits ist jedoch angesichts der immensen Kosten der weiteren Optimierung von Hits von größter kommerzieller Bedeutung für die pharmazeutische Industrie.

In einer bevorzugten Ausführungsform umfasst X ein Modul mit einer Spaltstelle $S_1$, welches durch die enzymkatalysierte Spaltreaktion in zwei, $X_1$ bzw. $X_2$ enthaltende Spaltprodukte definierter Molmasse spaltbar ist. Es wird jedoch in einer weiteren Ausführungsform auch ein Substrat und ein entsprechendes Detektionsverfahren bereitgestellt, bei dem X ein Modul mit mehreren Spaltstellen $S_1$ bis $S_n$ umfasst, wobei n > 1 ist. Durch die enzymatischen Spaltreaktionen entstehen somit n+1 Spaltprodukte definierter Molmasse. So kann das Substrat beispielsweise zwei Spaltsequenzen $S_1$ und $S_2$ aufweisen, welche durch die Enzyme $E_1$ und $E_2$ gespalten werden können. Dasselbe Substrat kann zur Inkubation mit jeweils einem der Enzyme $E_1$ oder $E_2$ verwendet werden. Die Entstehung der jeweiligen Produkte, Z-$X_1$ und $X_2X_3$-Y bzw. Z-$X_1X_2$ und $X_3$-Y, kann durch ein molmassensensitives Verfahren detektiert werden. Diese Ausführungsform des Substrates mit mehreren, insbesondere zwei, Spaltstellen, ermöglicht erstens mit nur einem Substrat mehrere Drug Discovery Programme durchzuführen (Suche nach Modulatoren von Enzym $E_1$ bzw. Enzym $E_2$) und zweitens die Selektivität von Modulatoren zu bestimmen. Hierbei wird die Fragestellung untersucht, ob ein Modulator

von Enzym $E_1$ auch ein Modulator von Enzym $E_2$ bzw. ob ein Modulator von Enzym $E_2$ auch ein Modulator von Enzym $E_1$ ist. Hierbei handelt es sich um eine wichtige Fragestellung in der Entwicklung pharmazeutischer Produkte, da etwaige unerwünschte Nebenwirkungen eines Modulators von $E_1$ auf weitere Enzyme wie z.B. $E_2$ erkannt werden können.

In einer bevorzugten Ausführungsform ist das Substrat derart ausgestaltet, dass die Molmasse von Z so gewählt ist, dass Z-$X_1$ bzw. $X_{n+1}$-Z mindestens etwa 60 % der Gesamtmolmasse des Substrates Z-X-Y bzw. Y-X-Z beträgt. Oftmals ist es jedoch bevorzugt, dass Z - $X_1$ bzw. $X_{n+1}$-Z mindestens etwa 70%, insbesondere mindestens etwa 80 %, besonders bevorzugt mindestens etwa 90 % der Gesamtmolmasse des Substrates beträgt. In Abhängigkeit von der Detektionseffizienz des verwendeten molmassensensitiven Detektionsverfahrens kann somit die optimale Molmassendifferenz oder molekulare Volumendifferenz zwischen Edukt und nach der enzymatischen Spaltreaktion entstandenen Produkten - eingestellt werden. So sind beispielsweise besonders geeignete Detektionsverfahren solche, die auf Fluoreszenzpolarisations- bzw. Fluoreszenzanisotropiebestimmungen beruhen, oder die Fluoreszenzkorrelationsspektroskopie. Die Detektion kann bevorzugt unter Verwendung eines konfokalen optischen Aufbaus durchgeführt werden, durch den aufgrund der sehr kleinen Meßvolumina (bis hinab in den Femtoliterbereich) etwaige Hintergrundsignale deutlich reduziert werden können. Mit dem erfindungsgemäßen Substrat, welches als Reportermodul Y somit bevorzugt einen Fluoreszenzmarker aufweist, können direkt oder indirekt eine Reihe molekularer Parameter ausgelesen werden. Die Korrelationszeit, d. h. die charakteristische Zeit, die ein Molekül benötigt, um eine definierte Bewegung auszuführen, ist direkt mit dem Volumen des Moleküls verbunden. Die Rotationskorrelationszeit $\phi$ ist gemäß folgender Beziehung gegeben

$$\phi = \eta V / RT$$

mit

- $\eta$: Viskosität
- V: Volumen des Moleküls
- R: allg. Gaskonstante
- T: Temperatur in K

Dies zeigt, dass unter typischen Assaybedingungen bei fixierter Temperatur in einem Lösungsmittel mit fester Viskosität $\phi = \eta \times$ Konstante gilt.

Der experimentell gut zugängliche Parameter r, die Anisotropie des Fluoreszenzlichtes, hängt gemäß der Perrin-Gleichung

$$r = r_0 / [1 + (\tau / \phi)]$$

mit $r_0$ Anisotropie zum Zeitpunkt t = 0
$\tau$ Fluoreszenzlebensdauer des Farbstoffs
mit der Rotationskorrelationszeit $\phi$ zusammen.

In dem erfindungsgemäßen Verfahren wird durch die Spaltreaktion (z. B. proteolytische Reaktion) eine Veränderung des Volumens bzw. der Molmasse der das farbstofftragende Reportermodul Y umfassenden chemischen Verbindung herbeigeführt. Dabei ist besonders zu erwähnen, dass die Anisotropie nicht nur als Summensignal auslesbar ist, sondern auch auf der Ebene einzelner Moleküle analysiert werden kann. Dies bedeutet, dass direkt aus der Probe heraus die absolute Konzentration von z. B. proteolytisch gespaltenem Substrat mit einer hohen Rotationskorrelationszeit (schnelle Rotation) und die absolute Konzentration von (noch) nicht reagiertem Substrat mit niedriger Korrelationszeit (langsame Rotation) bestimmbar ist. Eine externe Kalibration entfällt, da hier direkt das Verhältnis der beiden Spezies bestimmt und somit auf einfache Weise der Umsatz berechnet werden kann. Eine analoge Betrachtung gilt auch für das Verhalten des erfindungsgemäßen Substrates beim Einsatz von Detektionssystemen, die eine Translationsdiffusionskorrelationszeit ermitteln. So ist durch Fluoreszenzkorrelationsspektroskopie (FCS) die Diffusionskonstante des fluoreszenten Moleküles zu ermitteln, die in direkter Weise von der Masse des Moleküls abhängt und mit der ebenfalls eine absolute Konzentration der beiden Spezies detektierbar ist.

Um den inerten Charakter des bevorzugt polymeren Anteils Z am Gesamtsubstrat zu erzielen, wird erfindungsgemäß vorgeschlagen, Z und X aus unterschiedlichen Substanzklassen auszuwählen. So könnte beispielsweise eine proteolytische Enzymreaktion mittels molmassensensitiver optischer Verfahren untersucht werden, in denen X eine Aminosäuresequenz mit geeigneten Spaltstellen für das verwendete Enzym darstellt und Z einer anderen Substanzklasse angehört, d. h. kein Peptid bzw. Protein ist. In diesem Falle ist es besonders vorteilhaft, Z aus Nukleotiden aufzubauen, wobei insbesondere ein doppelsträngiger Nukleinsäurestrang bevorzugt ist. Ein derartiges Substrat ist beispielhaft in Figur 1 dargestellt. Aber auch jedes andere, bevorzugt hydrophile Polymer, eignet sich als Z Modul. Hydrophile Z-Module sind besonders bevorzugt, wenn der Assay in wässriger Lösung durchgeführt werden soll, um den biologischen Gegebenheiten Rechnung zu tragen.

Das erfindungsgemäße Substrat und seine Anwendung im erfindungsgemäßen Spaltassay zeigt im Gegensatz zu bereits bekannten Systemen die folgenden Vorteile:

1. Klar definierte Spaltprodukte nach der Spaltreaktion wie z. B. einer proteolytischen Reaktion: Es entstehen nur Spaltprodukte, die durch die Struktur des Substrates klar definiert sind und eindeutige Molmassen besitzen.
2. Homogene Signalverteilung aufgrund definierter Molekülstruktur: Da klar definierte Bruchstücke nach der Spaltreaktion wie z. B. einer proteolytischen Reaktion vorhanden sind, ist auch nur eine eindeutige Rotations- bzw. Diffusionskorrelationszeit für das farbstofftragende Bruchstück zu erwarten. Eine im Stand der Technik oftmals auftretende Polydispersität der Produkte mit einhergehender "Verschmierung" des detektierten Signals wird vermieden.
3. Modularer Aufbau: Das erfindungsgemäße Substrat weist einen modularen Aufbau auf und kann in vielfacher Weise an das zu untersuchende Problem angepasst werden:

- Reportermodul: Bei optischer Detektion kann aus einem weiten Bereich an kopplungsfähigen Farbstoffen (Aminreaktiv, Thio-reaktiv, Carbonsäure-reaktiv etc.) der detektionstechnisch, chemisch und photophysikalisch geeignete Farbstoff ausgewählt werden. So kann es sich hierbei beispielsweise um Coumarine, Fluoresceine, Rhodamine, Xanthene, Oxazine, Cyanine u. ä. sowie deren Derivate handeln.
- Modul X mit Spaltstelle: Jede bevorzugt synthetisch erzeugbare Spaltstelle kann eingebaut werden, d. h. jedes Enzym, wie z. B. eine Protease mit bekannter Erkennungs- bzw. Spaltsequenz, kann spezifisch und selektiv untersucht werden. Somit ist es möglich, in einem Gemisch unterschiedlicher Proteasen ein Substrat spezifisch für eine ausgewählte Protease anzubieten und spezifisch die Reaktion dieser Protease zu studieren.
- Das Modul Z (beispielsweise ein Polymer, insbesondere ein Oligonukleotid), kann frei gewählt werden und seine Länge / Masse kann nach den detektionstechnischen Vorgaben eingestellt werden. Auch sind Hybridisierungen an DNA-Sequenzen im Assay, falls gewünscht, möglich. Jedes einzelne Modul kann in sich unterschiedliche Substanzklassen aufweisen, z.B. kann das Modul Z eine Nukleinsäure sowie ein Cyclohexansäurederivat (siehe Ausführungsbeispiele) umfassen.

4. Linearer bzw. rigider Aufbau des Substrates: Durch die gestreckte Struktur eines z.B. in Figur 1 beschriebenen Substrates, im besonderen beim Einsatz des DNA-Doppelstranges, wird gewährleistet, dass es zu keiner Beeinflussung der enzymatischen, insbesondere proteolytischen Reaktion durch molekulare Wechselwirkung in Form von räumlichen Kontakten zwischen dem Z- und X-Modul (z.B. dem Oligonukleotid- und dem Peptid-Teil in Figur 1) kommt.
5. Die Wasserlöslichkeit kann selektiv eingestellt werden. So ist insbesondere eine hohe Wasserlöslichkeit durch Verwendung einer polyanionische Oligomer-Einheit als Z-Modul erzielbar. Für biologische Assaysysteme in wässriger Umgebung ist eine hohe Wasserlöslichkeit des Substrates naturgemäß notwendig und vorteilhaft.
6. Direktes Verfolgen des Reaktionverlaufes: Das Substrat gibt die Möglichkeit, die enzymatische Spaltreaktion während des Reaktionsablaufes zu beobachten und damit kinetische Studien zu betreiben.
7. Das Substrat ist per se detektierbar (vgl. Stand der Technik mit fluorogenen/chromogenen Prinzipien).
8. Das Modul Z kann so designed werden, dass es auch als (i) Erkennungsstelle für die Enzymbindung (z.B. Bindung von bestimmten Restriktionsendonukleasen) oder (ii) als Bindungsstelle für die Enzymbindung/-aktivität beeinflussende Faktoren dient.
9. Das Reportermodul Y kann sich räumlich getrennt von der Spaltregion befinden (vgl. Stand der Technik mit fluorogenem/chromogenem Prinzip; FRET).

Ferner betrifft die Erfindung in einem weiteren Aspekt ein Verfahren zur Detektion enzymkatalysierter Verknüpfungsreaktionen mit folgenden Schritten:

a) Bereitstellen folgender chemischer Verbindungen

$$Z - X_1 \text{ und } X_{n+1} - Y \text{ sowie optional } X_2 \text{ bis } X_n$$

oder

Y- $X_1$ und $X_{n+1}$ - Z sowie optional $X_2$ bis $X_n$

wobei Z ein Modul wählbarer Molmasse umfasst, welches bezüglich der enzymatischen Verknüpfungsreaktion inert ist,
$X_1$ einen Modulbaustein umfasst, welcher durch die enzymkatalysierte(n) Verknüpfungsreaktion(en) direkt mit $X_{n+1}$ im Fall n = 1 oder optional über mindestens einen Modulbaustein ausgewählt aus $X_2$ bis $X_n$ im Fall n > 1 indirekt verknüpfbar ist,
$X_{n+1}$ einen Modulbaustein umfasst, welcher durch die enzymkatalysierte(n) Verknüpfungsreaktion(en) direkt mit $X_1$ im Fall n = 1 oder optional über mindestens einen Modulbaustein ausgewählt aus $X_2$ bis $X_n$ im Fall n > 1 indirekt verknüpfbar ist,
Y ein Reportermodul umfasst,
n eine ganze Zahl ≥ 1 ist, und
die Molmasse von $X_{n+1}$-Y bzw. Y-$X_1$ höchstens fünfzig Prozent der Gesamtmolmasse des entstehenden Verknüpfungsproduktes beträgt;
als Substrate für das/die die Verknüpfungsreaktion katalysierende(n) Enzym(e);

b) Inkubieren der Verbindungen mit dem/den die Verknüpfungsreaktion(en) katalysierenden Enzym(en) unter Entstehung von Verknüpfungsprodukten

Z-X-Y

oder

Y-X-Z

wobei X die Modulbausteine $X_1$ und $X_{n+1}$ sowie optional mindestens einen Modulbaustein ausgewählt aus $X_2$ bis $X_n$ umfasst;

c) Detektieren der enzymatischen Aktivität(en) durch Bestimmung der/des das Reportermodul Y enthaltenden Verknüpfungsprodukte(s) mittels eines molmassensensitiven Detektions-verfahrens.

In einer bevorzugten Ausführungsform dieses Verfahrens werden zwei Modulbausteine $X_1$ und $X_2$ durch die enzymkatalysierte Reaktion direkt miteinander verknüpft. Es ist jedoch auch möglich, $X_1$ und $X_{n+1}$ durch einen Modulbaustein $X_2$ indirekt miteinander zu verknüpfen. Eine weitere Ausgestaltung des erfindungsgemäßen Verfahrens besteht darin, $X_1$ und $X_{n+1}$ durch eine Vielzahl von Bausteinen indirekt miteinander zu verknüpfen. Diese Bausteine werden ausgewählt aus den Bausteinen $X_2$ bis $X_n$.

Zur Durchführung enzymkatalysierter Verknüpfungsreaktionen wird erfindungsgemäß auch eine Anordnung folgender chemischer Verbindungen für das/die Verknüpfungsreaktion(en) katalysierende(n) Enzym(e) bereitgestellt:

Z - $X_1$ und $X_{n+1}$ - Y sowie optional $X_2$ bis $X_n$

oder

Y- $X_1$ und $X_{n+1}$ - Z sowie optional $X_2$ bis $X_n$

wobei $X_1$ einen Modulbaustein umfasst, welcher durch die enzymkatalysierte Verknüpfungsreaktion direkt mit $X_{n+1}$ im Fall n = 1 oder optional über mindestens einen Modulbaustein ausgewählt aus $X_2$ bis $X_n$ im Fall n > 1 indirekt verknüpfbar ist,
$X_{n+1}$ einen Modulbaustein umfasst, welcher durch die enzymkatalysierte Verknüpfungsreaktion direkt mit $X_1$ im Fall n = 1 oder optional über mindestens einen Modulbaustein ausgewählt aus $X_2$ bis $X_n$ im Fall n > 1 indirekt verknüpfbar ist,
Y ein Reportermodul umfasst,
n eine ganze Zahl ≥ 1 ist, und
Z ein Modul wählbarer Molmasse umfasst, welches bezüglich der enzymatischen Verknüpfungsreaktion inert ist, und dessen Molmasse derart gewählt ist, dass die Molmasse von $X_{n+1}$-Y bzw. V-$X_1$ höchstens fünfzig Prozent der Gesamtmolmasse des entstehenden Verknüpfungsproduktes beträgt,
sowie optional mindestens ein die Verknüpfungsreaktion katalysierendes Enzym, Co-Substrate, Co-Faktoren, Modulatoren, Puffer, und/oder Reagenzien zum Abstoppen der enzymatischen Reaktion.

Die Molmasse von Z ist bevorzugt - sowohl bezogen auf das oben genannte Verfahren zur Detektion von

Verknüpfungsreaktionen als auch auf die Beschreibung der Anordnung - derart gewählt, dass die Molmasse von V-$X_1$ bzw. $X_{n+1}$-Y höchstens etwa vierzig, insbesondere höchstens etwa dreißig, bevorzugt höchstens etwa zwanzig, besonders bevorzugt etwa zehn Prozent der Molmasse der Verbindung Z-X-Y bzw. Y-X-Z beträgt. Hierdurch läßt sich analog zu der Beschreibung des erfindungsgemäßen Verfahrens zur Detektion von Spaltreaktionen auch in Verknüpfungsreaktionen eine auf das Detektionsverfahren optimal ausgelegte Substratwahl ermöglichen.

Wie bereits bei der Beschreibung des erfindungsgemäßen Substrates für Spaltreaktionen erörtert, ist es auch bei diesen Substraten für Verknüpfungsreaktionen bevorzugt, dass X und Z unterschiedlichen Substanzklassen angehören. So können diese beispielsweise jeweils folgenden Substanzklassen angehören: Nukleinsäuren, Peptidnukleinsäuren (PNA), Peptide, Proteine, Lipide, Kohlenhydrate oder Derivate der vorgenannten Substanzen. Insbesondere kann es sich um synthetische Polymere handeln.

Generell sei angemerkt, dass alle aufgeführten Substrate, Kits und Assays besonders geeignet sind zur Testung von Enzym- und/oder Modulatorenspezifitäten, zur Testung von Enzym- und/oder Modulatorenaktivitäten, zur Modulatoren- und/oder Substratidentifizierung, zur Detektion von Kontaminationen in chemischen oder biologischen Proben, zum Screening nach pharmakologisch wirksamen Substanzen oder auch zu diagnostischen Zwecken. Diese Applikationen werden insbesondere auch aus den unten dargestellten Figuren und zugehörigen Figurenbeschreibungen deutlich.

Im allgemeinen ist ferner auch wünschenswert, Z so zu wählen, dass dieses Modul eine derartige chemische Struktur aufweist, dass es sterisch nicht mit der Spalt- oder Verknüpfungsstelle interagiert. Dies kann insbesondere durch eine rigide Struktur von Z ermöglicht werden, z.B. eine lineare Struktur wie die eines Polyanions. Wie bereits an einigen Stellen erwähnt, ist es besonders bevorzugt, hier eine doppelsträngige Nukleinsäure bzw. ein Derivat hiervon zu verwenden.

In einer Ausführungsform kann es sich bei dem die Reaktion katalysierenden Enzym um eine Hydrolase handeln, insbesondere eine Lipase, Phosphatase, Amylase, Glycosidase, Nucleosidase, Peptidase, Protease, Amidase, Pyrophosphatase, ATPase, oder auch Phosphoamidase. Das die Reaktion katalysierende Enzym kann auch eine Lyase sein, insbesondere eine C-C-, C-O-, C-N- oder C-S-Lyase. Hierbei kann es sich insbesondere um eine (De-)Carboxylase, Aldolase, Dehydratase, Ammoniak-Lyase, Arginosuccinase oder Cysteindesulfhydrase handeln. Ferner kann das die Reaktion katalysierende Enzym beispielsweise eine Ligase sein, insbesondere eine C-O-, C-S-, C-N- oder C-C-Ligase. Beispielhaft seien hier eine aminosäure-aktivierende Ligase, Acyl-CoA-Synthetase, Glutaminsynthetase oder Pyruvatcarboxylase genannt. Zudem können auch Transferasen wie DNA- oder RNA-Polymerasen eingesetzt werden. Im allgemeinen kann es bevorzugt sein, Enzyme mit Substrat-, Gruppen-, oder sterischer Spezifität einzusetzen. Wie in den Ausführungsbeispielen ausgeführt, kann es sich bei dem Enzym insbesondere auch um eine Caspase handeln, wie z. B. Caspase 3 oder Caspase 8. Das erfindungsgemäße Substrat umfasst dann in seinem mit X bezeichneten Modul eine für die jeweilige Caspase spezifische Spaltsequenz, während das mit Z bezeichnete Modul bevorzugt einer anderen Substanzklasse, wie z. B. den Oligonukleotiden, angehört.

Insbesondere kann die enzymkatalysierte Spaltung- oder Verknüpfungsreaktion in Gegenwart eines oder mehrerer Co-Substrate oder Cofaktoren durchgeführt werden. Im übrigen können ein oder mehrere Substrate und/oder Substrate gleichzeitig oder sequentiell eingesetzt werden. Die Verknüpfungs- bzw. Spaltreaktionen können insbesondere in Gegenwart von Modulatoren oder potentiellen Modulatoren der enzymatischen Aktivität durchgeführt werden.

Es ist insbesondere bevorzugt, die erfindungsgemäßen Verfahren als homogene Assays, d.h. ohne Waschschritte, durchzuführen. Insbesondere die oben näher beschriebenen fluoreszenz-spektroskopischen Detektionsverfahren erlauben in Kombination mit den erfindungsgemäßen Substraten für Spalt- oder Verknüpfungsreaktionen ein derartiges Vorgehen. Um die Resultate nicht zu verfälschen, mag es im Einzelfall wünschenswert sein, das die Spalt- bzw. Verknüpfungsreaktion katalysierende Enzym vorzugsweise (bio-)chemisch von dem das Reportermodul Y enthaltenden Reaktionsprodukt zu lösen. Ferner ist es im allgemeinen oftmals vorteilhaft, ein Reportermodul Y mit einem Fluoreszenzfarbstoff zu verwenden.

Die Erfindung wird nachfolgend beispielhaft durch die Figuren verdeutlicht. Ferner werden Ausführungsbeispiele, insbesondere zur Synthese der erfindungsgemäßen Substrate, dargestellt.

Figur 1 zeigt den Aufbau einer Ausführungsform des erfindungsgemäßen Substrates, wobei es sich bei dem Fluoreszenzfarbstoff um jeden kovalent an eine Aminosäure koppelbaren Farbstoff handeln kann, insbesondere um Coumarine, Fluoresceine, Rhodamine, Xanthene, Oxazine, Cyanine u. ä. oder Derivate hiervon. Die Länge des Peptidmoduls ist bevorzugt von n = 1 bis n = 100, besonders bevorzugt bis n=10. Innerhalb der Peptidsequenz ist vorzugsweise mindestens eine Kombination von aufeinanderfolgenden Aminosäuren vorhanden, wie sie spezifisch für eine bestimmte proteolytische Reaktion ist. Die Erkennungssequenz der jeweiligen Protease kann hier ebenfalls selektiv und spezifisch eingebaut werden. Das nach der proteolytischen Spaltung entstehende, den DNA-Doppelstrang tragende Bruchstück ist bevorzugt so beschaffen, dass sein Anteil am Gesamtmolekulargewicht des Moleküls mindestens 80 % ausmacht.

Anhand des der Figur 2 zugrundeliegenden Beispiels soll demonstriert werden, wie eine Ausführungsform des erfindungsgemäßen Substrats, welches die spezifische Erkennungssequenz der Protease Caspase 3 trägt, proteolytisch

gespalten wird. Die proteolytische Spaltung wird durch Messung der Diffusionszeit $\tau_D$ mittels Fluoreszenzkorrelationsspektroskopie (FCS) verfolgt. Dabei wurde eine 5 nM Lösung des in Ausführungsbeispiel 1 beschriebenen Substrates in Puffer (50 mM HEPES pH 7,5, 100 mM NaCl, 10% Saccharose, 0.1% CHAPS, 10 mM DTT) zusammen mit 1 nM Caspase inkubiert und der Verlauf des Fluoreszenzkorrelationssignals zeitabhängig beobachtet. Die Anpassung der gemessenen Kurve an die Korrelationsfunktion (2-Komponenten) erfolgte mit den Werten $\tau_{D1}$ = 313 ns (ungespaltenes Substrat) und $\tau_{D2}$ = 70 µs (gespaltenes Substrat). Bereits in der Veränderung der Korrelationskurven mit der Zeit ist der Abfall der mittleren Diffusionszeit erkennbar.

In der genauen Analyse mit den unter Figur 2 genannten Parametern ergeben sich die in Figur 3 aufgetragenen Anteile der einzelnen Diffusionszeiten und damit der Anteil der einzelnen Spezies farbstofftragender Partikel.

Anhand des Figur 4 zugrundeliegenden Beispiels soll demonstriert werden, wie eine Ausführungsform des erfindungsgemäßen Substrats, das die spezifische Erkennungssequenz der Protease Caspase 3 trägt, proteolytisch gespalten wird. Die proteolytische Spaltung wird durch Messung der Fluoreszenzpolarisation verfolgt. Dabei wurde eine 1 nM Lösung des in Ausführungsbeispiel 1 beschriebenen Substrates in Puffer (50 mM HEPES pH 7,5, 100 mM NaCl, 10% Saccharose, 0.1% CHAPS, 10 mM DTT) zusammen mit 1 nM Caspase inkubiert und der Verlauf der Fluoreszenzpolarisation zeitabhängig beobachtet. Die Grafik zeigt, wie die Polarisation der Probe mit der Zeit abnimmt. Die angegebenen Standardabweichungen ergeben sich aus jeweils 25 Messungen. Die Ursache für diesen Verlauf liegt in der Spaltung des Substrates durch die proteolytische Aktivität der Caspase.

Figur 5 zeigt eine Ausführungsform des erfindungsgemäßen Substrates und seine Anwendung in einem Caspase 3 Assay. Das tripartite Substrat besteht aus einem schematisch dargestellten Polymer, einer die für Caspase 3 typischen Erkennungssequenz DEVD (im Einbuchstabencode) enthaltenden zu spaltenden Sequenz und dem Fluoreszenzfarbstoff TAMRA. Es zeigt sich ein mit der Spaltung einhergehender deutlicher Unterschied in der detektierten Fluoreszenzpolarisation.

Figur 6 zeigt die Bestimmung des Z' Faktors, welcher ein Maß für das Signal-Rausch-Verhältnis des Assays darstellt, unter Verwendung von 10 nM Substrat und Anwendung eines konfokalen Detektionsaufbaus. Das Experiment liefert dem Assayentwickler eine Aussage über (i) den dynamischen Bereich, (ii) das Zeitfenster für eine lineare Reaktion, (iii) Standardabweichung, CV (Datenstatistik) und (iv) Z' Faktor (Vergleich mit inhibierter Reaktion). Diese Ergebnisse erlauben die Bestimmung des sog. "Screening windows" (könnte dieser Assay gescreent werden). Es zeigt sich hier ein exzellenter Z' Faktor von ≥ 0.83 nach bereits 40 Minuten.

Figur 7 zeigt die ermittelten $IC_{50}$ Werte. Die Abbildung zeigt, dass eine nahezu identische Dose-response-Kurve in Ab-/Anwesenheit von DMSO gemessen werden kann. Da Compounds in der Regel in DMSO gelöst sind und im HTS eine Restkonzentration an DMSO oft unvermeidbar ist, ist ein derartiger Test wichtig.

Figur 8 verdeutlicht die Anpassung des Assays auf 1 µl Endvolumen in Nanotiterplatten. Es zeigt sich, dass die sogenannten "Highs" (in Anwesenheit eines Inhibitors) und "Lows" (in Abwesenheit eines Inhibitors) immer noch gut unterscheidbar und der Z' Faktor ausreichend ist, um den Assay in einem Hochdurchsatzscreening nach Modulatoren der enzymatischen Aktivität, die ggf. später als Pharmazeutika Anwendung finden können, anzuwenden.

Figur 9 zeigt den Vergleich der $IC_{50}$ Werte in einem 60 µl und einem 1 µl Assayformat unter Zusatz von Screening Additiven. Die Assayperformance im 1 µl Format, welches in vorteilhafter Weise aufgrund des nur geringen Substanzverbrauches im Hochdurchsatzscreening Anwendung findet, ist vergleichbar zur Performance in der Assayentwicklung. Der Assayentwickler kann somit den Assay für einen Hochdurchsatzscreen nach pharmakologisch aktiven Substanzen freigeben.

Figur 10 zeigt schematisch eine Ausführungsform des erfindungsgemäßen Substrates (Z-X-Y) und seine Anwendung in der Detektion enzymkatalysierter Spaltreaktionen. Das Substrat weist in dieser Abbildung eine einzige Spaltstelle $S_1$ auf. Bei der Spaltung durch das Enzym $E_1$ entstehen die beiden Spaltprodukte Z-$X_1$ und $X_2$-Y. Diese Reaktion kann durch ein molmassensensitives Verfahren detektiert werden.

Figur 11 zeigt schematisch eine Ausführungsform des erfindungsgemäßen Substrates (Z-X-Y) und seine Anwendung zur Identifizierung von Modulatoren (Compounds "C") enzymkatalysierter Spaltreaktionen ("drug discovery"). Das Substrat weist in dieser Abbildung eine einzige Spaltstelle $S_1$ auf. Compounds, welche die enzymatische Aktivität modulieren (inhibieren oder aktivieren), führen zu einer geringeren Menge (im Falle einer Inhibierung) oder zu einer größeren Menge (im Falle einer Aktivierung) an enstehenden Produkten innerhalb einer definierten Inkubationszeit. Durch ein molmassensensitives Verfahren kann dann die Menge an entstehenden Produkten in Gegenwart einer Compound (diese Abbildung) ins Verhältnis gesetzt werden zur Menge an entstehenden Produkten in Abwesenheit einer Compound (siehe Figur 10), um den Grad der Inhibition bzw. der Aktivierung der enzymatischen Reaktion zu ermitteln. In dieser Abbildung inhibiert die Compound C das Enzym E1 völlig, so dass keine Produkte Z-$X_1$ und $X_2$-Y entstehen. Dies entspricht einem Inhibitionsgrad von 100 %.

Figur 12 zeigt eine weitere Ausführungsform des erfindungsgemäßen Substrates (Z-X-Y) und seine Anwendung in der Detektion sequentiell durchgeführter enzymkatalysierter Spaltreaktionen. Das Substrat weist in dieser Abbildung zwei Spaltsequenzen $S_1$ und $S_2$ auf. Dasselbe Substrat kann durch Inkubation mit einem der Enzyme $E_1$ oder $E_2$, welches das Substrat an der Spaltstelle $S_1$ bzw. $S_2$ spaltet, eingesetzt werden. Es entstehen die Produkte Z-$X_1$ und

$X_2X_3$-Y bzw. Z-$X_1X_2$ und $X_3$-Y; die Reaktion kann durch ein molmassensensitives Verfahren detektiert werden. Diese Ausführung des Substrates mit mehreren Spaltstellen ermöglicht erstens mit nur einem Substrat mehrere Drug Discovery Programme durchzuführen (Suche nach Modulatoren von Enzym $E_1$ bzw. Enzym $E_2$) und zweitens die Selektivität von Modulatoren zu bestimmen (Fragestellung: ist ein Modulator von Enzym E1 auch ein Modulator von Enzym $E_2$ bzw. ist ein Modulator von Enzym $E_2$ auch ein Modulator von Enzym $E_1$?).

Figur 13 zeigt eine Ausführungsform des erfindungsgemäßen Substrates (Z-X-Y) und eine weitere Anwendung in der Detektion gleichzeitig durchgeführter enzymkatalysierter Spalt-reaktionen. Das Substrat weist in dieser Abbildung zwei Spaltsequenzen $S_1$ und $S_2$ auf; der Assay kann jedoch auch mit mehr als zwei Spaltsequenzen sowie einer korrespondierenden Anzahl Enzyme durchgeführt werden. Das Substrat kann durch gleichzeitige Inkubation mit den beiden Enzymen $E_1$ und $E_2$ in die Spaltprodukte Z-$X_1$, $X_2$ und $X_3$-Y gespalten werden. Dieser Assay kann dazu eingesetzt werden, in einer Drug Discovery Kampagne Modulatoren einer gesamten Enzymklasse zu identifizieren (z.B. Modulatoren von Caspasen), um zum Beispiel ein Breitband-Medikament zu entwickeln.

Figur 14 zeigt eine Ausführungsform des erfindungsgemäßen Substrates (Z-X-Y) und seine Anwendung zur Detektion von Kontaminationen K, welche zur Spaltung des Moduls X führen. Das Substrat weist in dieser Abbildung eine einzige Spaltsequenz $S_1$ auf, welche bei der Inkubation mit Enzym $E_1$ gespalten würde. Das Modul Z ist erfindungsgemäß gegenüber der enzymatischen Reaktion von E1 inert. Inkubiert man jedoch das Substrat nicht mit dem Enzym $E_1$ sondern mit einer beliebigen Probe, so läßt sich durch ein molmassensensitives Verfahren detektieren, ob das Substrat dennoch gespalten wird. Dabei können gegebenenfalls mehr Produkte entstehen (unspezifische Kontamination), als wenn man mit dem Enzym $E_1$ spalten würde. In dieser Abbildung führt die Kontamination K zu den drei Produkten Z-$X_1$, $X_2$ und $X_3$-Y. Setzt man dem Inkubationsansatz zusätzlich Inhibitoren hinzu, welche die Spaltung von Z verhindern, so kann man gezielt Kontaminationen identifizieren, welche X spalten. Über das molmassensensitive Verfahren lassen sich auch spezifische Kontaminationen detektieren.

Figur 15 zeigt eine Ausführungsform des erfindungsgemäßen Substrates (Z-X-Y) und seine Anwendung zur Detektion von Kontaminationen K, welche zur Spaltung des Moduls Z führen. Das Substrat weist in dieser Abbildung eine einzige Spaltsequenz $S_1$ auf, welche bei der Inkubation mit dem Enzym $E_1$ gespalten würde. Das Modul Z ist erfindungsgemäß gegenüber der enzymatischen Reaktion von E1 inert. Inkubiert man jedoch das Substrat nicht mit dem Enzym $E_1$ sondern mit einer beliebigen Probe, so läßt sich durch ein molmassensensitives Verfahren detektieren, ob das Substrat dennoch gespalten wird. In dieser Abbildung führt die Kontamination K zu den drei Produkten $Z_1$, $Z_2$, $Z_3$-X-Y. Setzt man dem Inkubationsansatz zusätzlich Inhibitoren hinzu, welche die Spaltung von X verhindern, so kann man gezielt Kontaminationen identifizieren, welche Z spalten.

Figur 16 zeigt eine Ausführungsform des erfindungsgemäßen Substrates (Z-X-Y) und seine Anwendung zur Detektion von Kontaminationen K der zugesetzten Charge des Enzyms $E_1$, welches das Substrat an der Spaltstelle $S_1$ spaltet. Inkubiert man das Substrat mit dem Enzym $E_1$ und zusätzlich mit einem Inhibitor $I_1$ der enzymatischen Aktivität des Enzyms $E_1$, so kann man durch ein molmassensensitives Verfahren detektieren, ob dennoch eine Spaltung des Substrates erfolgt. In dieser Abbildung entstehen die drei Produkte $Z_1$, $Z_2$, $Z_3$-X-Y.

Figur 17 zeigt schematisch zwei Ausführungsformen des erfindungsgemäßen Substrates (Z-X-Y) und deren Anwendung zur Bestimmung der Substratspezifität des Enzyms $E_1$. In der ersten Ausführungsform des erfindungsgemäßen Substrates (Z-X-Y) enthält das Modul X die Spaltstelle $S_1$. In der zweiten Ausführungsform des erfindungsgemäßen Substrates (Z-X-Y) enthält das Modul X die Spaltstelle $S_2$. Die beiden Substrate enthalten unterschiedliche Reportermodule $Y_1$ bzw. $Y_2$. Insbesondere können $Y_1$ und $Y_2$ unterschiedliche Fluoreszenzfarbstoffe sein, welche bei unterscheidbaren Wellenlängen Fluoreszenzlicht emittieren. Inkubiert man beide Substrate gleichzeitig mit dem Enzym $E_1$, so kann man durch ein molmassensensitives Verfahren, welches die beiden Reportermodule unterscheiden kann, detektieren, (1) ob eines der beiden Substrate gespalten wurde, (2) welches der beiden Substrate gespalten wurde und (3) ob beide Substrate gespalten wurden. In dieser Abbildung ist $E_1$ spezifisch für das Substrat mit der Spaltstelle $S_1$.

Figur 18 zeigt die Detektion der Aktivität eines einzelne Substrate verknüpfenden Enzyms E durch Bestimmung des das Reportermodul Y enthaltenden Verknüpfungsproduktes Z-X-Y mittels eines molmassensensitiven Detektionsverfahrens. Auch dieser Assay läßt sich zur Identifizierung von Modulatoren der Enzymaktivität einsetzen. In dieser Abbildung werden Z-$X_1$ und $X_3$-Y indirekt über $X_2$ miteinander verknüpft.

Figur 19 zeigt die praktische Anwendung des in Figur 5 beschriebenen Substrates zur Identifizierung von Caspase-3-Inhibitoren in einer Drug Discovery Kampagne. Die Abbildung gibt einen Überblick über den Ablauf und die Ergebnisse der Kampagne. 193146 potentielle Modulatoren (Compounds) wurden auf ihre inhibitorische Wirkung auf Caspase-3 hin analysiert. Die Compounds wurden dabei in einer Konzentration von 17 μM eingesetzt. 1.5 % (primäre Hit-Rate) der Compounds zeigten dabei eine Inhibierung, welche einen definierten Schwellenwert überschritt. Diese primären Hits wurden in dergleichen Konzentration (17 μM) in Mehrfachbestimmungen nochmals auf ihre inhibitorische Wirkung hin getestet. Die inhibitorische Wirkung von 229 Compounds konnte auf diese Weise bestätigt werden. Die Potenz dieser 229 Compounds wurde über die Aufnahme von Dosis-Wirkungs-Kurven ermittelt, welche die Abhängigkeit der inhibitorischen Wirkung einer Compound von der eingesetzten Konzentration dieser Compound widerspiegeln. 27 Compounds zeigten dabei einen $IC_{50}$-Wert, der kleiner als 50 μM ist. Der mittlere Wert für den Z'-Faktor, welcher ein

Maß für die Güte eines Assays darstellt (Zhang, J.-H., et al. (1999). J. Biomol. Screen., 4, 67-73), betrug über den gesamten Prozeß 0.8. Z' Werte größer als 0.5 zeigen an, dass ein exzellenter Assay vorliegt. Somit ist der Caspase-3 Assay sehr geeignet für eine Drug Discovery Kampagne.

Figur 20 zeigt beispielhaft die Dosis-Wirkungs-Kurve einer der 27 in der Legende zu Figur 19 beschriebenen inhibitorischen Compounds. Die Compound wurde in 6 unterschiedlichen Konzentrationen (50 μM, 25 μM, 12.5 μM, 6.25 μM, 3.13 μM und 1.56 μM) auf ihre inhibitorische Wirkung hin untersucht. Es wurde ein $IC_{50}$-Wert von 19.11 μM bestimmt.

__**Ausführungsbeispiel 1**__

**Synthese eines Caspase 3 - spezifischen Substrates (Peptid Nr. 1):**

Das Peptid mit der Sequenz **H-Gly-Asp-Glu-Val-Asp-Gly-Lys-OH** wurde nach Standardfestphasensynthese (Fmoc-Strategie) hergestellt. Als Harz diente Rink Amide MBHA resin mit der Beladung 0,54 mmol/g. Es wurde eine Ansatzgröße von 20 μmol gewählt ( $m_{Harz}$ = 37,0mg). Die Aktivierung der Aminosäuren erfolgte mit 5 Äquivalenten HATU ([O-(7-azabenzotriazol-1-yl)-1,1,3,3-tetramethyluronium hexafluorophosphate]) und 10 Äquivalenten DIPEA (N-Ethyl-diisopropylamin) in DMF (N,N-Dimethylformamide) (100 μmol HATU (38,0mg) und 200 μmol DIPEA (34,0 μl). Das Volumen des Lösungsmittels betrug 5 ml/g Harz. Es wurden 5 Äquivalente Aminosäure eingesetzt. Die Reaktionszeit betrug 2 mal 1 Stunde (Doppelkopplung) bei Raumtemperatur. Die Abspaltung der Fmoc-Schutzgruppe (Fluorenylmethoxycarbonyl) wurde mit 20 % Piperidin in DMF durchgeführt. Die Abspaltungszeit betrug 2 mal 15 Minuten. Jeweils nach den Kopplungen, als auch nach der Fmoc-Abspaltung, wurde das Harz mit 3x DMF, 2x DMF/DCM (Dichlormethan) [1:1], 3x DMF p.a. gewaschen. Die Fmoc-Schutzgruppe am N-terminus wurde nicht entfernt. Somit ergibt sich nach den Kopplungen von Fmoc-Lys(Mtt)-OH, Fmoc-Gly-OH, Fmoc-Asp(OtBu)-OH, Fmoc-Val-OH, Fmoc-Glu(OtBu)-OH, Fmoc-Asp(OtBu)-OH und Fmoc-Gly-OH folgende Sequenz: **Fmoc-Gly-Asp(OtBu)-Glu(OtBu)-Val-Asp(OtBu)-Gly-Lys (Mtt)-Harz.**

**Kopplung des Farbstoffes an das Peptid:**

Für die Markierung wurden 5 μmol peptidbeladenes Harz (9,3 mg) eingesetzt. Von dem immobilisierten, vollständig geschützten Fmoc-GDEVDGK-OH wurde die Mtt(4-Methyltrityl)-Schutzgruppe am Lysin mit 1 ml 30% HFIP (1,1,1,3,3,3-Hexafluoro-2-propanol) in DMF entfernt. Es wurde noch 6x mit je 1 ml 30% HFIP in DMF nachgewaschen. Das anschließende Waschen erfolgte wiederum mit 3x DMF, 2x DMF/DCM, 3x DMF pA. Das gebildete primäre Amin wurde mit 1 Äquivalent (bezogen auf die Ansatzgröße → 5μmol) 5-TAMRA-NHS (5-Carboxytetramethylrhodamine, succinimidylester) in DMF gekoppelt. Die Reaktionszeit betrug 8 Stunden bei Raumtemperatur. Nach der Markierung wurde das Harz gewaschen. Es ergibt sich nun folgendes Zwischenprodukt:

**Fmoc-Peptid 1-5-TAMRA**
**MW: 1352 g/mol**

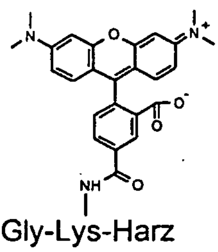

Fmoc-Gly-Asp(OtBu)-Glu(OtBu)-Val-Asp(OtBu)-Gly-Lys-Harz

**Kopplung eines Linkers an das markierte Peptid:**

Nach der Fmoc-Abspaltung mit 20% Piperidin in DMF wurde das markierte Peptid 1 mit einem Linker (4-(N-Maleimidomethyl)-cyclohexane-1-carboxylic acid N-hydroxy-succinimide ester) zum entsprechenden Maleimid modifiziert. Für die Kopplung des Linkers wurden 2μmol Peptidharz (m=3,7mg) eingesetzt. Es wurden 2 Äquivalente Linker (bezogen auf die Ansatzgröße) in DMF auf das Harz gegeben. Die Reaktionszeit betrug 8 Stunden bei Raumtemperatur. Nach dem Waschen des Harzes mit 3x DMF, 3x DMF/DCM (1:1), 3x DCM, 3x tert-Butylmethylether wurde das Zwischenprodukt mit TFA/TIPS/$H_2O$ (95%/2,5%/2,5%) vom Harz abgespalten. Die Abspaltungszeit betrug 2,5 Stunden bei Raumtemperatur. So ergibt sich folgendes Zwischenprodukt:

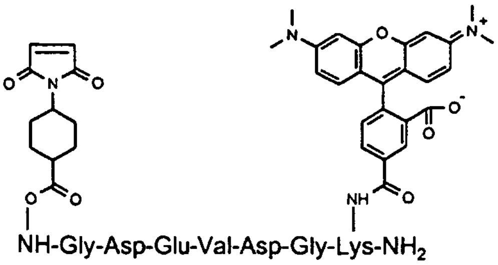

**Maleimid-Peptid1-5-TAMRA**
**MW: 1349 Da**

Die Reinigung des Zwischenproduktes erfolgte mittels HPLC. Die verwendeten Laufmittel waren Wasser + 0,1% TFA (A) und Methanol (B). Verwendet wurde eine Säule (Waters, Symmetry 100, RP-18, 5 μm, 150mm × 19mm) mit einem Fluß von 18 ml/min. Gereinigt wurde mit folgendem Gradienten: 0min → 10% B, 5min → 30% B, 45min → 70% B, 50min → 100% B. Die Charakterisierung des Zwischenproduktes wurde mittels LC-MS, MALDI-TOF-MS und UV/VIS-Spektroskopie vorgenommen.

**Kopplung des Maleimid-Peptid1-5-TAMRA an DNA:**

Das entstandene Zwischenprodukt wurde mit dem lyophilisierten DNA-Doppelstrang, der an einem Strang 5'-thiomodifiziert wurde, zum gewünschten Endprodukt umgesetzt.

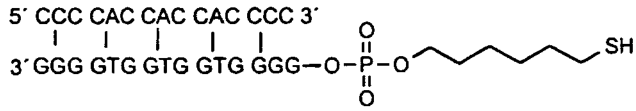

**DNA, doppelsträngig 5'-Thiol modifiziert**
**MW: 11625 g/mol**

Dazu wurde das Maleimid-Peptid1-5-TAMRA (42 nmol) in 50 μl 10mM Natriumphosphatpuffer pH 7,5 aufgenommen. Die Anzahl der Äquivalente des Peptides bezogen auf die DNA beträgt 4. Demzufolge wurden 10,4 nmol DNA in 10 μl $H_2O$ eingesetzt. Die Reaktionszeit betrug 5 Stunden bei 25°C.

**DNA-Maleimid-Peptid1-5-TAMRA**
**MW: 12956 g/mol**

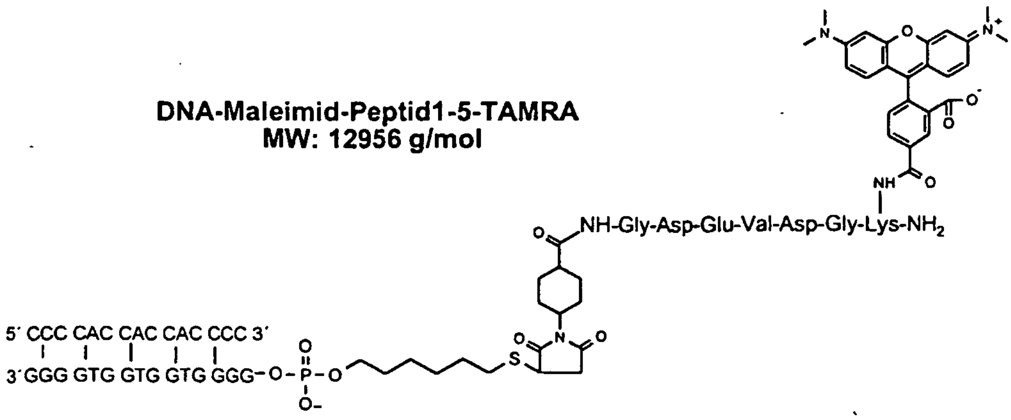

Gereinigt wurde das Produkt mittels HPLC über eine Gelsäule (Phenomenex, Biosep-SEC-S 2000, 300mm × 4,6mm) mit einem Fluß von 0,15 ml/min bei 12°C. Es wurde ein isokratischer Lauf mit 10mM Natriumphosphatpuffer pH 7,5 über 45 Minuten durchgeführt. Die Charakterisierung des Produktes erfolgte mittels UV/VIS-Spektroskopie, Fluoreszenzspektroskopie, Polarisations- und Anisotropiebestimmung. Die Reinheit wurde über einen analytischen Lauf

über die Gelsäule ermittelt.

Das für Caspase 3 spezifische Substrat umfasst somit: Asp-Glu-Val-Asp*-Gly, wobei Asp-Glu-Val-Asp die Erkennungssequenz der Protease darstellt und die Spaltung nach Asp* erfolgt.

**<u>Ausführungsbeispiel 2</u>**

**Synthese eines Caspase 8 - spezifischen Substrates (Peptid Nr. 2):**

Peptid mit der Sequenz **H-Gly-Ile-Glu-Thr-Asp-Gly-Lys-OH** wurde nach Standardfestphasensynthese (Fmoc-Strategie) hergestellt. Als Harz diente Rink Amide MBHA resin mit der Beladung 0,54 mmol/g. Es wurde eine Ansatzgröße-von 20 μmol gewählt (37,0mg). Die Aktivierung der Aminosäuren erfolgte mit 5 Äquivalenten HATU ([O-(7-azabenzotriazol-1-yl)-1,1,3,3-tetramethyluronium hexafluorophosphate] und 10 Äquivalenten DIPEA (N-Ethyl-diisopropylamin) in DMF (N,N-Dimethylformamide). Somit ergeben sich folgende Mengen pro Kopplung: 100 μmol HATU (38,0mg) und 200 μmol DIPEA (34,0μl). Das Volumen des Lösungsmittels betrug 5 ml/g Harz. Es wurden 5 Äquivalente Aminosäure eingesetzt. Die Reaktionszeit betrug 2 mal 1 Stunde (Doppelkopplung) bei Raumtemperatur. Die Abspaltung der Fmoc-Schutzgruppe (Fluorenylmethoxycarbonyl) wurde mit 20% Piperidin in DMF durchgeführt. Die Abspaltungszeit betrug 2 mal 15 Minuten. Jeweils nach den Kopplungen, als auch nach der Fmoc-Abspaltung, wurde das Harz mit 3x DMF, 2x DMF/DCM (Dichlormethan) [1:1], 3x DMF p.a. gewaschen. Die Fmoc-Schutzgruppe am N-terminus wurde nicht entfernt. Somit ergab sich nach den Kopplungen von Fmoc-Lys(Mtt)-OH, Fmoc-Gly-OH, Fmoc-Asp(OtBu)-OH, Fmoc-Thr (tBu)-OH, Fmoc-Glu(OtBu)-OH, Fmoc-Ile-OH und Fmoc-Gly-OH folgende Sequenz: **Fmoc-Gly-Ile-Glu(OtBu)-Thr (tBu)-Asp(OtBu)-Gly-Lys(Mtt)-Harz.**

**Kopplung des Farbstoffes an Peptid 2:**

Für die Markierung wurden 5 μmol peptidbeladenens Harz (9,3mg) eingesetzt. Vom immobilisierten, vollständig geschützten Fmoc-GIETDGK-OH wurde die Mtt(4-Methyltrityl) -Schutzgruppe am Lysin mit 1 ml 30% HFIP (1,1,1,3,3,3-Hexafluoro-2-propanol) in DMF entfernt. Es wurde noch 6 mal mit je 1 ml 30% HFIP in DMF nachgewaschen. Das anschließende Waschen erfolgte wiederum mit 3x DMF, 2x DMF/DCM, 3x DMF pA. Das gebildete primäre Amin wurde mit 1 Äquivalent (bezogen auf die Ansatzgröße → 5μmol) 5-TAMRA-NHS (5-carboxytetramethylrhodamine, succinimidylester) in DMF gekoppelt. Die Reaktionszeit betrug etwa 8 Stunden (über Nacht) bei Raumtemperatur. Es ergab sich nun folgendes Zwischenprodukt:

**Fmoc-Peptid 2-5-TAMRA**
**MW: 1352 g/mol**

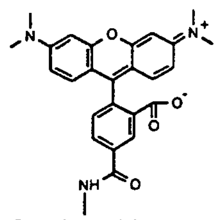

Fmoc-Gly-Ile-Glu(OtBu)-Thr(tBu)-Asp(OtBu)-Gly-Lys-Harz

**Kopplung eines Linkers an das markierte Peptid 2:**

Nach der Fmoc-Abspaltung mit 20% Piperidin in DMF wurde das Peptid 2 mit einem Linker (4-(N-Maleimidomethyl)-cyclohexane-1-carboxylic acid N-hydroxy-succinimide ester) zum entsprechenden Maleimid modifiziert. Für die Kopplung des Linkers wurden 2μmol Peptidharz (m=3,7mg) eingesetzt. Es wurden 2 Äquivalente Linker (bezogen auf die Ansatzgröße) in DMF auf das Harz gegeben. Die Reaktionszeit betrug 8 Stunden bei Raumtemperatur. Nach dem Waschen des Harzes mit 3x DMF, 3x DMF/DCM (1:1), 3x DCM, 3x tert-Butylmethylether wurde das Zwischenprodukt mit TFA/TIPS/$H_2O$ (95%/2,5%/2,5%) vom Harz abgespalten. Die Abspaltungszeit betrug 2,5 Stunden bei Raumtemperatur. So ergab sich folgendes Zwischenprodukt:

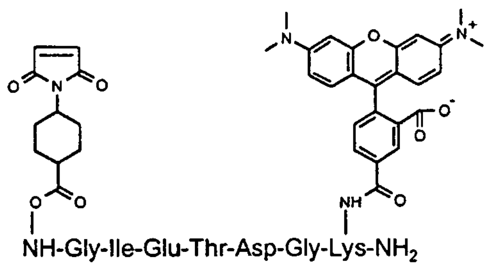

**Maleimid-Peptid2-5-TAMRA**
**MW: 1349 Da**

Die Reinigung des Zwischenproduktes erfolgte mittels HPLC. Die verwendeten Laufmittel waren Wasser + 0,1% TFA (A) und Methanol (B). Verwendet wurde eine Säule (Waters, Symmetry 100, RP-18, 5 μm, 150mm x 19mm) mit einem Fluss von 18 ml/min. Gereinigt wurde mit folgendem Gradienten: 0min → 10% B, 5min → 30% B, 45min → 70% B, 50min → 100% B. Die Charakterisierung des Zwischenproduktes wurde mittels LC-MS, MALDI-TOF-MS und UV/VIS-Spektroskopie vorgenommen.

**Kopplung des Maleimid-Peptid 2-5-TAMRA an DNA:**

Das entstandene Zwischenprodukt wurde mit dem lyophilisierten 5'-thiomodifizierten DNA-Doppelstrang zum gewünschten Endprodukt umgesetzt.

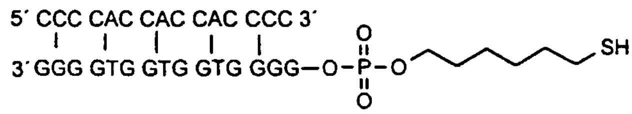

**DNA, doppelsträngig 5'-Thiol modifiziert**
**MW: 11625 g/mol**

Dazu wurde das Maleimid-Peptid 2-5-TAMRA (n = 42 nmol) in 50 μl 10mM Natriumphosphatpuffer pH 7,5 aufgenommen. Die Anzahl der Äquivalente des Peptides bezogen auf die DNA beträgt 4. Demzufolge wurden 10,4 nmol DNA in 10 μl $H_2O$ eingesetzt. Die Reaktionszeit betrug 5 Stunden bei 25°C. Dadurch entstand folgendes Endprodukt:

**DNA-Maleimid-Peptid 2-5-TAMRA**
**MW: 12956 g/mol**

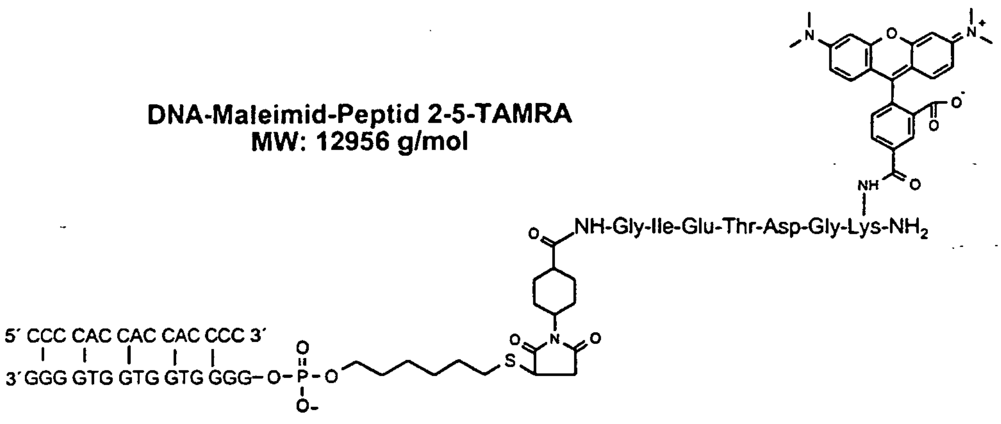

Gereinigt wurde das Produkt mittels HPLC über eine Gelsäule (Phenomenex, Biosep-SEC-S 2000, 300mm x 4,6mm) mit einem Fluss von 0,15 ml/min bei 12°C. Es wurde ein isokratischer Lauf mit 10mM Natriumphosphatpuffer pH 7,5 über 45 Minuten durchgeführt. Die Charakterisierung des Produktes erfolgte mittels UV/VIS-Spektroskopie, Floureszensspektroskopie, Polarisations- und Anisotropiebestimmung. Die Reinheit wurde über einen analytischen Lauf über die Gelsäule ermittelt.

Das für Caspase 8 spezifische Substrat umfasst somit: Ile-Glu-Thr-Asp*-Gly, wobei Ile-Glu-Thr-Asp die Erkennungssequenz der Protease darstellt und die Spaltung nach Asp* erfolgt.

## Ausführungsbeispiel 3

### Ultra High-Throughput Screening (uHTS) nach Inhibitoren zur Identifizierung von Inhibitoren der Cystein-Protease Caspase-3 ("drug discovery"):

Apoptose (programmierter Zelltod, PCD) ist ein Phänomen, welches man unter physiologischen und pathologischen Bedingungen beobachten kann. Apoptose stellt die Hauptform des Zelltods eukaryontischer Organismen dar. Sie tritt unter anderem bei der Entwicklung eines Embryos und Metamorphose-Prozessen auf. Bei bestimmten Krankheiten (Alzheimer, Tumoren, Autoimmunkrankheiten) ist der Apoptose-Prozess gestört. Schlüsselmoleküle in der apoptotischen Kaskade sind die so genannten Caspasen. Inhibitoren oder Aktivatoren der Caspasen wären daher wertvoll, um Patienten zu behandeln, welche unter einer Fehlregulation der Apoptose leiden.

Die Cystein-Protease Caspase-3 ist ein zentraler Mediator von Apoptose in einer Vielzahl unterschiedlicher Zellen. Ein selektiver Caspase-3 Inhibitor könnte zur Therapie, beispielsweise bei Schlaganfällen, bei Organtransplantationen, bei Parkinson Krankheit und amyothropher Lateralsclerose (ALS), eingesetzt werden.

Das in Ausführungsbeispiel 1 beschriebene Substrat wurde daher auch für "ultra high-throughput screening" (uHTS) nach Inhibitoren zur Identifizierung von Inhibitoren von Caspase-3 ("drug discovery") erfolgreich eingesetzt. Die Ausführung und die Resultate dieser Screening Kampagne sind in der Legende zu Figur 19 näher beschrieben.

## Ausführungsbeispiel 4

### Selektivitätsbestimmung von im Ausführungsbeispiel 3 beschriebenen Caspase-3 Inhibitoren:

Das in Ausführungsbeispiel 2 beschriebene Caspase-8 Substrat wurde zur Bestimmung der Selektivität der im ultra high-throughput screening (uHTS) identifizierten Inhibitoren von Caspase-3 eingesetzt. Das bereits für Caspase-3 angewendete Verfahren zur molmassensensitiven Detektion einer enzymkatalysierten Spaltreaktion konnte hierbei erfolgreich auch in einem Caspase-8 Assay eingesetzt werden.

## Patentansprüche

**1.** Verfahren zur Detektion enzymkatalysierter Spaltreaktionen mit folgenden Schritten:

a) Bereitstellen einer modularen chemischen Verbindung enthaltend das folgende Sequenzmotiv

$$Z - X - Y \text{ oder } Y - X - Z$$

wobei Z ein Modul wählbarer Molmasse umfasst, welches bezüglich der enzymatischen Spaltreaktion inert ist, X ein Modul mit n Spaltstellen $S_1$ bis $S_n$ umfasst, welches durch die enzymkatalysierte Spaltreaktion in mindestens zwei, $X_1$ bzw.
$X_{n+1}$ enthaltende Spaltprodukte definierter Molmasse spaltbar ist, und n eine ganze Zahl ≥ 1 darstellt,
Y ein fluoreszentes Reportermodul umfasst,
und wobei die Bestandteile der Verbindungen Z - X - Y oder Y- X - Z kovalent miteinander verbunden sind als Substrat für das/die die Spaltreaktion(en) katalysierende(n) Enzyme;
b) Inkubieren der Verbindung mit dem/den die Spaltreaktion(en) katalysierenden Enzym(en) unter Entstehung mindestens zweier Spaltprodukte, welche enthalten

$$Z - X_1 \text{ und } X_{n+1} - Y$$

oder

$$Y - X_1 \text{ und } X_{n+1} - Z$$

wobei die Molmasse des Z - $X_1$ bzw. $X_{n+1}$- Z enthaltenden Spaltproduktes mindestens fünfzig Prozent der Gesamtmolmasse des Substrates beträgt;
c) Detektieren der enzymatischen Aktivität durch Bestimmung des das Reportermodul Y enthaltenden Spaltproduktes mittels eines molmassensensitiven Verfahrens, welches auf bevorzugt konfokaler Fluoreszenzkorrelationsspektroskopie, Fluoreszenzpolarisationsbestimmung und/oder Fluoreszenzanisotropiebestimmung beruht.

**2.** Verfahren zur Detektion enzymkatalysierter Verknüpfungsreaktionen mit folgenden Schritten:

a) Bereitstellen folgender chemischer Verbindungen

$Z - X_1$ und $X_{n+1} - Y$ sowie optional $X_2$ bis $X_n$

oder

$Y- X_1$ und $X_{n+1} - Z$ sowie optional $X_2$ bis $X_n$

wobei Z ein Modul wählbarer Molmasse umfasst, welches bezüglich der enzymatischen Verknüpfungsreaktion inert ist,
$X_1$ einen Modulbaustein umfasst, welcher durch die enzymkatalysierte(n) Verknüpfungsreaktion(en) direkt mit $X_{n+1}$ im Fall n = 1 oder optional über mindestens einen Modulbaustein ausgewählt aus $X_2$ bis $X_n$ im Fall n > 1 indirekt verknüpfbar ist,
$X_{n+1}$ einen Modulbaustein umfasst, welcher durch die enzymkatalysierte(n) Verknüpfungsreaktion(en) direkt mit $X_1$ im Fall n = 1 oder optional über mindestens einen Modulbaustein ausgewählt aus $X_2$ bis $X_n$ im Fall n > 1 indirekt verknüpfbar ist,
Y ein fluoreszentes Reportermodul umfasst,
n eine ganze Zahl ≥ 1 ist,
die Molmasse von $X_{n+1}$-Y bzw. Y-$X_1$ höchstens fünfzig Prozent der Gesamtmolmasse des entstehenden Verknüpfungsproduktes beträgt und die Bestandteile der einzelnen Substrate der enzymatischen Verküpfungsreaktion(en) kovalent miteinander verbunden sind
als Substrate für das/die die Verknüpfungsreaktion katalysierende(n) Enzym(e);
b) Inkubieren der Verbindungen mit dem/den die Verknüpfungsreaktion(en) katalysierenden Enzym(en) unter Entstehung von Verknüpfungsprodukten

Z - X - Y

oder

Y - X - Z

wobei X die Modulbausteine $X_1$ und $X_{n+1}$ sowie optional mindestens einen Modulbaustein ausgewählt aus $X_2$ bis $X_n$ umfasst;
c) Detektieren der enzymatischen Aktivität(en) durch Bestimmung des das Reportermodul Y enthaltenden Verknüpfungsproduktes mittels eines molmassensensitiven Detektionsverfahrens, welches auf bevorzugt konfokaler Fluoreszenzkorrelationsspektroskopie, Fluoreszenzpolarisationsbestimmung und/oder Fluoreszenzanisotropiebestimmung beruht.

**3.** Verfahren nach Anspruch 1, **dadurch gekennzeichnet, dass** im Spaltassay die Molmasse von $Z - X_1$ bzw. $X_{n+1}$ - Z mindestens etwa sechzig, insbesondere mindestens etwa siebzig, bevorzugt mindestens etwa achtzig, besonders bevorzugt mindestens etwa neunzig Prozent der Molmasse der Verbindung Z - X - Y bzw. Y- X - Z beträgt.

**4.** Verfahren nach Anspruch 2, **dadurch gekennzeichnet, dass** im Verknüpfungsassay die Molmasse von Y-$X_1$ bzw. $X_{n+1}$-Y höchstens etwa vierzig, insbesondere höchstens etwa dreißig, bevorzugt höchstens etwa zwanzig, besonders bevorzugt höchstens etwa zehn Prozent der Molmasse der Verbindung Z -X - Y bzw. Y - X - Z beträgt.

**5.** Verfahren nach einem der Ansprüche 1 bis 4, **dadurch gekennzeichnet, dass** Z ein aus Monomerbausteinen aufgebautes Polymer umfasst.

**6.** Verfahren nach einem der Ansprüche 1 bis 5, **dadurch gekennzeichnet, dass** X und Z unterschiedlichen Substanzklassen angehören, die vorzugsweise ausgewählt werden aus der Gruppe umfassend Synthetische Polymere, Dendrimere, Naturstoffe, Nukleinsäuren, Peptidnukleinsäuren (PNA), Peptide, Proteine, Lipide, Kohlenhydrate oder

Derivate der vorgenannten Substanzen, und insbesondere, falls Z eine Nukleinsäure ist, es eine doppelsträngige Nukleinsäure oder ein doppelsträngiges Nukleinsäurederivat umfasst.

**7.** Verfahren nach einem der Ansprüche 1 bis 6, **dadurch gekennzeichnet, dass** das Detektionsverfahren ein Maß für die Rotations- oder Translationsdiffusionsgeschwindigkeit des das Reportermodul Y enthaltenden Spalt- oder Verknüpfungsproduktes bestimmt.

**8.** Verfahren nach einem der Ansprüche 1 bis 7, **dadurch gekennzeichnet, dass** das Verfahren als homogener Assay durchgeführt wird.

**9.** Verfahren nach einem der Ansprüche 1 bis 8, **dadurch gekennzeichnet, dass** ein oder mehrere Enzyme und/oder Substrate gleichzeitig oder sequentiell eingesetzt werden.

**10.** Verfahren nach einem der Ansprüche 1 bis 9, **dadurch gekennzeichnet, dass** es in Gegenwart von Modulatoren oder potentiellen Modulatoren der enzymatischen Aktivität durchgeführt wird.

**11.** Verfahren nach einem der Ansprüche 1 bis 10, **dadurch gekennzeichnet, dass** es zur Testung von Enzym- und/oder Modulatorenspezifitäten, zur Testung von Enzym- und/oder Modulatorenaktivitäten, zur Modulatoren- und/oder Substratidentifizierung, zum Screening nach pharmakologisch wirksamen Substanzen, zu diagnostischen Zwecken oder zur Detektion von Kontaminationen in chemischen oder biologischen Proben eingesetzt wird, wobei insbesondere die eingesetzten Enzyme Substrat-, Gruppen- oder sterische Spezifität haben.

**12.** Verfahren nach einem der Ansprüche 1 bis 11, **dadurch gekennzeichnet, dass** Z eine rigide, vorzugsweise lineare Struktur, aufweist.

## Claims

**1.** A method for the detection of enzyme-catalyzed cleavage reactions, comprising the following steps:

a) providing a modular chemical compound containing the following sequence motif:

$$Z - X - Y \text{ or } Y - X - Z$$

wherein Z comprises a module with a selectable molecular weight which is inert with respect to said enzymatic cleavage reaction;
X comprises a module with n cleavage sites $S_1$ to $S_n$ which can be cleaved by said enzyme-catalyzed cleavage reaction into at least two cleavage products containing $X_1$ and $X_{n+1}$, respectively, and having defined molecular weights, n being an integer of ≥ 1;
Y comprises a reporter module;
and wherein the components of compound Z - X - Y or Y - X - Z are covalently linked with each other;
as a substrate for said enzyme(s) catalyzing said cleavage reaction(s);
b) incubating the compound with said enzyme(s) catalyzing said cleavage reaction(s) to form at least two cleavage products which contain

$$Z - X_1 \text{ and } X_{n+1} - Y$$

or

$$Y - X_1 \text{ and } X_{n+1} - Z$$

wherein the molecular weight of the cleavage product containing Z-$X_1$ or $X_{n+1}$-Z is at least fifty percent of the total molecular weight of the substrate
c) detecting the enzymatic activity by determining the cleavage product containing the reporter module Y by means of a molecular-weight-sensitive method which is based on fluorescence correlation spectroscopy, preferably confocal fluorescence correlation spectroscopy, fluorescence polarization determination and/or fluorescence anisotropy determination.

**2.** A method for the detection of enzyme-catalyzed linking reactions, comprising the following steps:

a) providing the following chemical compounds:

$$Z - X_1 \text{ and } X_{n+1} - Y \text{ and optionally } X_2 \text{ to } X_n;$$

or

$$Y - X_1 \text{ and } X_{n+1} - Z \text{ and optionally } X_2 \text{ to } X_n;$$

wherein Z comprises a module with a selectable molecular weight which is inert with respect to said enzymatic linking reaction;
$X_1$ comprises a modular building block which can be linked by said enzyme-catalyzed linking reaction(s) directly with $X_{n+1}$ in the case where n = 1, or optionally indirectly through at least one modular building block selected from $X_2$ to $X_n$ in the case where n > 1;
$X_{n+1}$ comprises a modular building block which can be linked by said enzyme-catalyzed linking reaction(s) directly with $X_1$ in the case where n = 1, or optionally indirectly through at least one modular building block selected from $X_2$ to $X_n$ in the case where n > 1;
Y comprises a fluorescent reporter module;
n is an integer of ≥ 1;
the molecular weight of $X_{n+1}$-Y or Y-$X_1$ is at most fifty percent of the total molecular weight of the linked product formed;
and the components of the individual substrates of the enzymatic linking reaction(s) are covalently linked with each other;
as substrates for said enzyme(s) catalyzing said linking reaction;
b) incubating the compound with said enzyme(s) catalyzing said linking reaction(s) to form linked products

$$Z - X - Y$$

or

$$Y - X - Z$$

wherein X comprises the modular building blocks $X_1$ and $X_{n+1}$ and optionally at least one modular building block selected from $X_2$ to $X_n$;
c) detecting the enzymatic activity or activities by determining the linked product containing the reporter module Y by means of a molecular-weight-sensitive detection method which is based on fluorescence correlation spectroscopy, preferably confocal fluorescence correlation spectroscopy, fluorescence polarization determination and/or fluorescence anisotropy determination.

**3.** The method according to claim 1, **characterized in that** the molecular weight of Z-$X_1$ and $X_{n+1}$-Z in the cleavage assay is at least about sixty percent, especially at least about seventy percent, preferably at least about eighty percent, more preferably at least about ninety percent, of the molecular weight ofcompound Z - X - Y or Y - X - Z.

**4.** The method according to claim 2, **characterized in that** the molecular weight of Y-$X_1$ and $X_{n+1}$-Y in the linking assay is at most about forty percent, especially at most about thirty percent, preferably at most about twenty percent, more preferably at most about ten percent, of the molecular weight of compound Z - X - Y or Y - X - Z.

**5.** The method according to any of claims 1 to 4, **characterized in that** Z comprises a polymer constituted of monomer building blocks.

**6.** The method according to any of claims 1 to 5, **characterized in that** X and Z belong to different classes of substances, which are preferably selected from the group comprising synthetic polymers, dendrimers, natural substances, nucleic acids, peptide nucleic acids (PNA), peptides, proteins, lipids, carbohydrates or derivatives of the above mentioned substances, and in particular, if Z is a nucleic acid, it comprises a double-stranded nucleic acid or a double-stranded nucleic acid derivative.

**7.** The method according to any of claims 1 to 6, **characterized in that** the detection method determines a measure

of the rotational or translational diffusion rate of the cleavage or linked product containing said reporter module Y.

**8.** The method according to any of claims 1 to 7, **characterized in that** the said method is performed as a homogeneous assay.

**9.** The method according to any of claims 1 to 8, **characterized in that** one or more enzymes and/or substrates are employed simultaneously or sequentially.

**10.** The method according to any of claims 1 to 9, **characterized by** being performed in the presence of modulators or potential modulators of enzymatic activity.

**11.** The method according to any of claims 1 to 10, **characterized by** being employed for testing enzyme and/or modulator specificities, for testing enzyme and/or modulator activities, for the identification of modulators and/or substrates, for the screening for pharmacologically active substances, for diagnostic purposes, or for the detection of contaminations in chemical or biological samples, wherein, in particular, the enzymes employed have substrate, group or steric specificity.

**12.** The method according to any of claims 1 to 11, **characterized in that** Z has a rigid structure, preferably a linear structure.

**Revendications**

**1.** Procédé de détection de réactions de clivage à catalyse enzymatique comprenant les étapes suivantes consistant à :

a) fournir un composé chimique modulaire contenant le motif de séquence suivant

Z-X-Y

ou

Y-X-Z

dans lequel Z comprend un module de masse molaire sélectionnable, lequel est inerte par rapport à la réaction de clivage enzymatique,
X comprend un module présentant n sites de clivage $S_1$ à $S_n$, module qui, au moyen de la réaction de clivage à catalyse enzymatique, est clivable en au moins deux produits de clivage de masse molaire définie contenant $X_1$ ou $X_{n+1}$, et n représente un nombre entier ≥ 1,
Y comprend un module rapporteur fluorescent, et dans lequel les composants des composés Z-X-Y ou Y-X-Z sont liés les uns aux autres par une liaison covalente
en tant que substrat pour la ou les enzymes destinées à catalyser la ou les réactions de clivage ;
b) incuber le composé avec la ou les enzymes catalysant la ou les réactions de clivage avec formation d'au moins deux produits de clivage, lesquels contiennent

Z-$X_1$ et $X_{n+1}$-Y

ou

Y-$X_1$ et $X_{n+1}$-Z

dans lesquels la masse molaire du produit de clivage contenant Z-$X_1$ ou $X_{n+1}$-Z correspond à au moins cinquante pour cent de la masse molaire totale du substrat ;
c) détecter l'activité enzymatique en identifiant le produit de clivage contenant le module rapporteur Y au moyen d'un procédé sensible à la masse molaire, lequel repose sur une spectroscopie de corrélation de fluorescence de préférence à foyer commun, une étude de la polarisation de fluorescence et/ou une étude de l'anisotropie de fluorescence.

**2.** Procédé de détection de réactions de liaison à catalyse enzymatique comprenant les étapes suivantes consistant à :

a) fournir les composés chimiques suivants

$Z\text{-}X_1$ et $X_{n+1}\text{-}Y$ et éventuellement $X_2$ à $X_n$

ou

$Y\text{-}X_1$ et $X_{n+1}\text{-}Z$ et éventuellement $X_2$ à $X_n$

dans lequel Z comprend un module de masse molaire sélectionnable, lequel est inerte par rapport à la réaction de liaison enzymatique,
$X_1$ comprend un élément modulaire qui peut être lié, au moyen de la ou des réactions de liaison à catalyse enzymatique, directement à $X_{n+1}$ dans le cas où n = 1, ou éventuellement indirectement par l'intermédiaire d'au moins un élément modulaire choisi parmi $X_2$ à $X_n$ dans le cas où n > 1,
$X_{n+1}$ comprend un élément modulaire qui peut être lié, au moyen de la ou des réactions de liaison à catalyse enzymatique, directement à $X_1$ dans le cas où n - 1, ou éventuellement indirectement par l'intermédiaire d'au moins un élément modulaire choisi parmi $X_2$ à $X_n$ dans le cas où n > 1,
Y comprend un module rapporteur fluorescent,
n représente un nombre entier ≥ 1,
la masse molaire de $X_{n+1}\text{-}Y$ ou $Y\text{-}X_1$ correspond à au maximum cinquante pour cent de la masse molaire totale des produits de liaison formés et les composants des substrats individuels de la ou des réactions de liaison enzymatiques sont liés les uns aux autres de une liaison covalente
en tant que substrats pour la ou les enzymes catalysant la ou les réactions de liaison ;
b) incuber les composés avec la ou les enzymes catalysant la ou les réactions de liaison avec formation de produits de liaison

Z-X-Y

ou

Y-X-Z

dans lesquels X comprend les éléments modulaires $X_1$ et $X_{n+1}$ et éventuellement au moins un élément modulaire choisi parmi $X_2$ à $X_n$ ;
c) détecter la ou les activités enzymatiques en identifiant le produit de liaison contenant le module rapporteur Y au moyen d'un procédé de détection sensible à la masse molaire, lequel repose sur une spectroscopie de corrélation de fluorescence de préférence à foyer commun, une étude de la polarisation de fluorescence et/ou une étude de l'anisotropie de fluorescence.

**3.** Procédé selon la revendication 1, **caractérisé en ce que**, dans le dosage de clivage, la masse molaire de Z-X1 ou $X_{n+1}\text{-}Z$ correspond à au moins environ soixante, en particulier à au moins environ soixante-dix, de préférence à au moins environ quatre-vingt, de manière plus particulièrement préférée à au moins environ quatre-vingt-dix pour cent de la masse molaire du composé Z-X-Y ou Y-X-Z.

**4.** Procédé selon la revendication 2, **caractérisé en ce que**, dans le dosage de liaison , la masse molaire de Y-X1 ou $X_{n+1}\text{-}Y$ correspond à au maximum environ quarante, en particulier à au maximum environ trente, de préférence à au maximum environ vingt, de manière plus particulièrement préférée à au maximum environ dix pour cent de la masse molaire du composé Z-X-Y ou Y-X-Z.

**5.** Procédé selon l'une quelconque des revendications 1 à 4, **caractérisé en ce que** Z comprend un polymère formé à partir d'éléments monomères.

**6.** Procédé selon l'une quelconque des revendications 1 à 5, **caractérisé en ce que** X et Z appartiennent à des classes de substance différentes, choisies de préférence parmi le groupe constitué par les polymères synthétiques, les dendrimères, les substances naturelles, les acides nucléiques, les acides nucléiques peptidiques (ANP), les peptides, les protéines, les lipides, les glucides ou les dérivés des substances susmentionnées, et en particulier, dans le cas où Z est un acide nucléique, il comprend un acide nucléique double-brin ou un dérivé à double-brin d'un acide nucléique.

7. Procédé selon l'une quelconque des revendications 1 à 6, **caractérisé en ce que** le procédé de détection détermine une mesure de la vitesse de diffusion par rotation ou translation du produit de clivage ou de liaison contenant le module rapporteur Y.

8. Procédé selon l'une quelconque des revendications 1 à 7, **caractérisé en ce que** le procédé est réalisé en tant que dosage homogène.

9. Procédé selon l'une quelconque des revendications 1 à 8, **caractérisé en ce que** une ou plusieurs enzymes et/ou substrats sont mis en oeuvre simultanément ou de façon séquentielle.

10. Procédé selon l'une quelconque des revendications 1 à 9, **caractérisé en ce que** le procédé est réalisé en présence de modulateurs ou de modulateurs potentiels de l'activité enzymatique.

11. Procédé selon l'une quelconque des revendications 1 à 10, **caractérisé en ce que** le procédé est utilisé pour tester les spécificités d'enzymes et/ou de modulateurs, pour tester les activités d'enzymes et/ou de modulateurs, pour identifier des substrats et/ou des modulateurs, pour cribler des substances efficaces sur le plan pharmacologique, à des fins diagnostiques ou pour la détection de contaminations dans des échantillons chimiques ou biologiques, dans lequel les enzymes utilisées présentent en particulier une spécificité de substrat, de groupe ou une spécificité stérique.

12. Procédé selon l'une quelconque des revendications 1 à 11, **caractérisé en ce que** Z présente une structure rigide, de préférence linéaire.

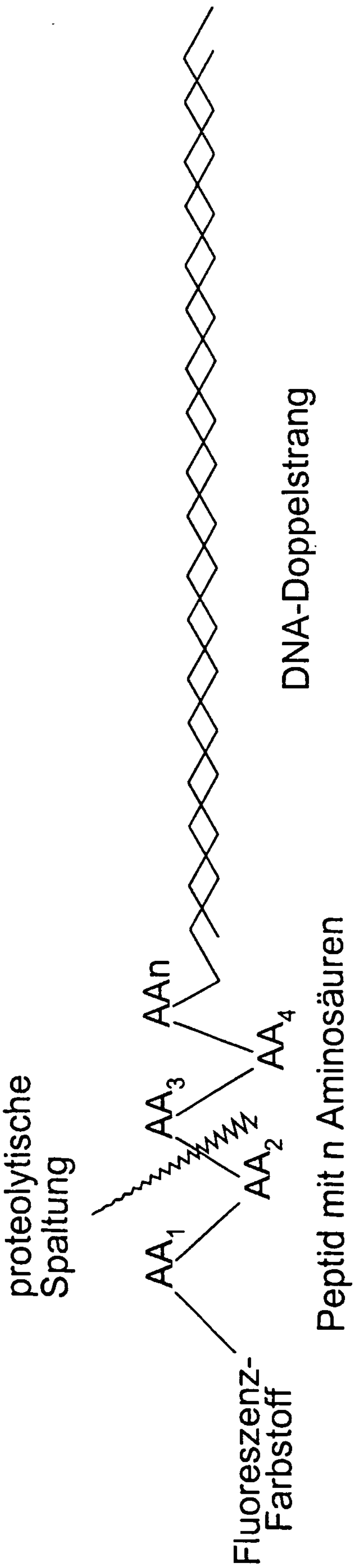
proteolytische Spaltung
Fluoreszenz-Farbstoff
$AA_1$
$AA_2$
$AA_3$
$AA_4$
AAn
Peptid mit n Aminosäuren
DNA-Doppelstrang

Fig.1

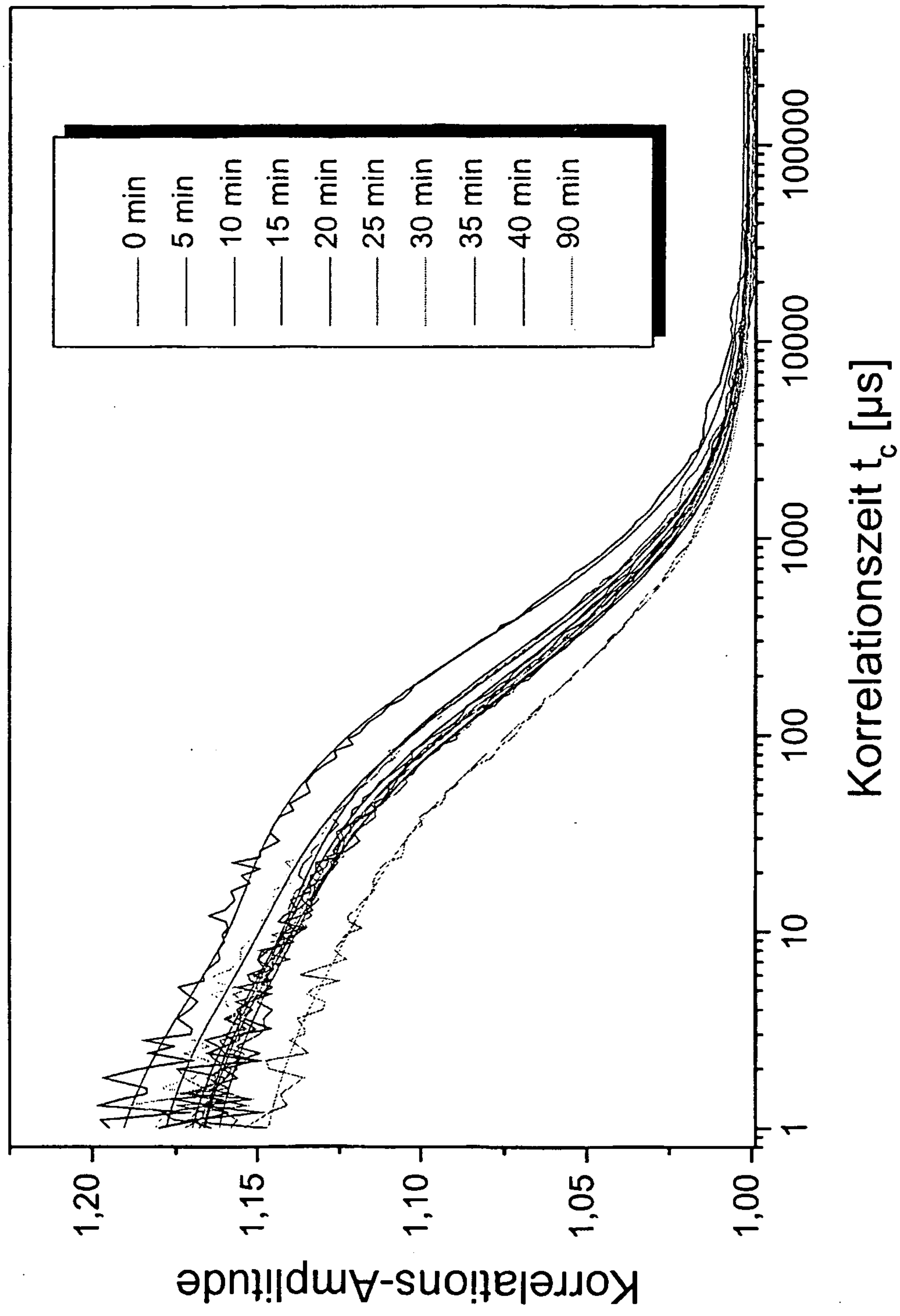
Korrelations-Amplitude
1,20
1,15
1,10
1,05
1,00
Korrelationszeit $t_c$ [µs]
1
10
100
1000
10000
100000
0 min
5 min
10 min
15 min
20 min
25 min
30 min
35 min
40 min
90 min

Fig.2

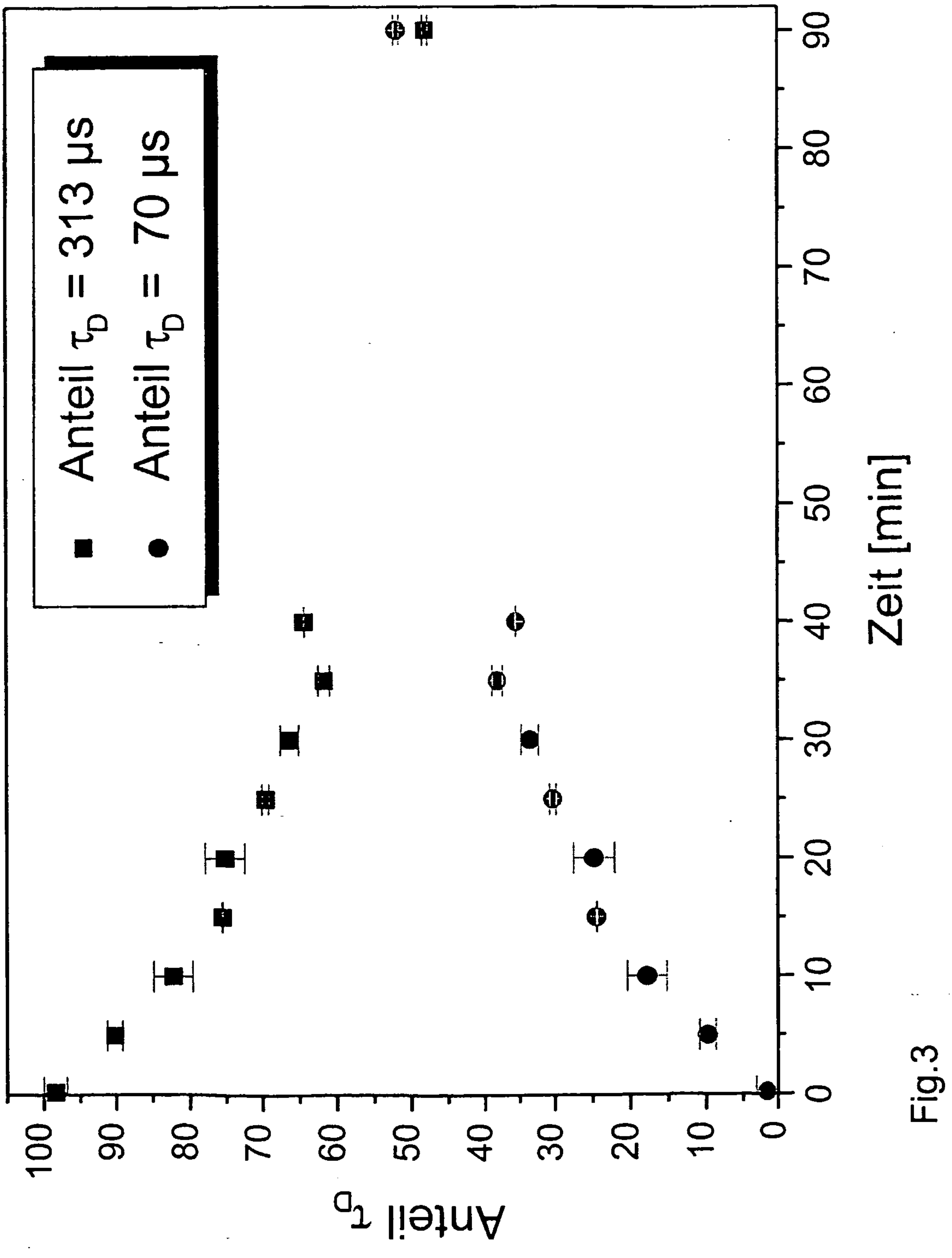
Anteil $\tau_D$ = 313 µs
Anteil $\tau_D$ = 70 µs
Anteil $\tau_D$
Zeit [min]
0
10
20
30
40
50
60
70
80
90
100

Fig.3

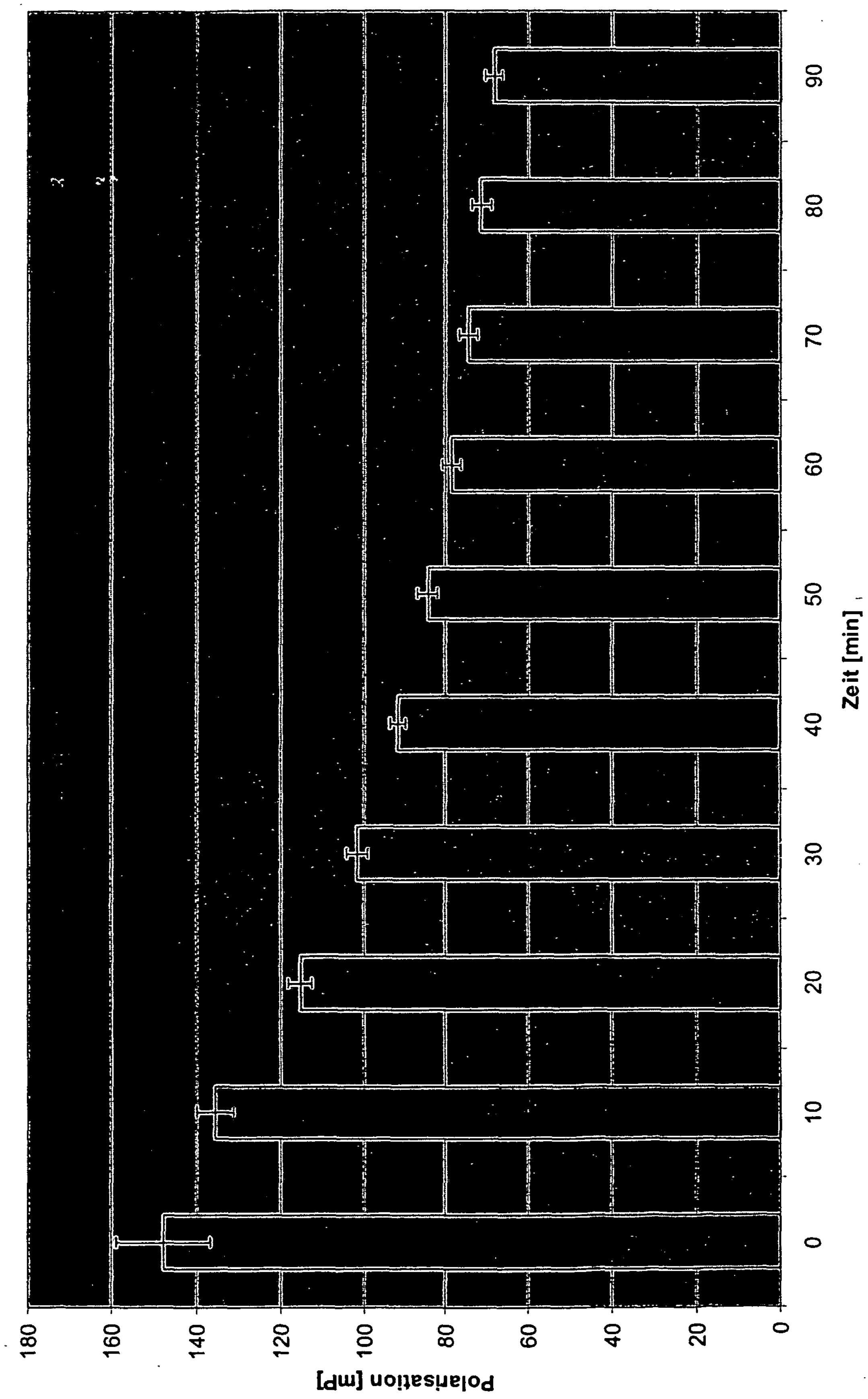
Polarisation [mP]
180
160
140
120
100
80
60
40
20
0
Zeit [min]
0
10
20
30
40
50
60
70
80
90

Fig.4

## Caspase-3 Assay

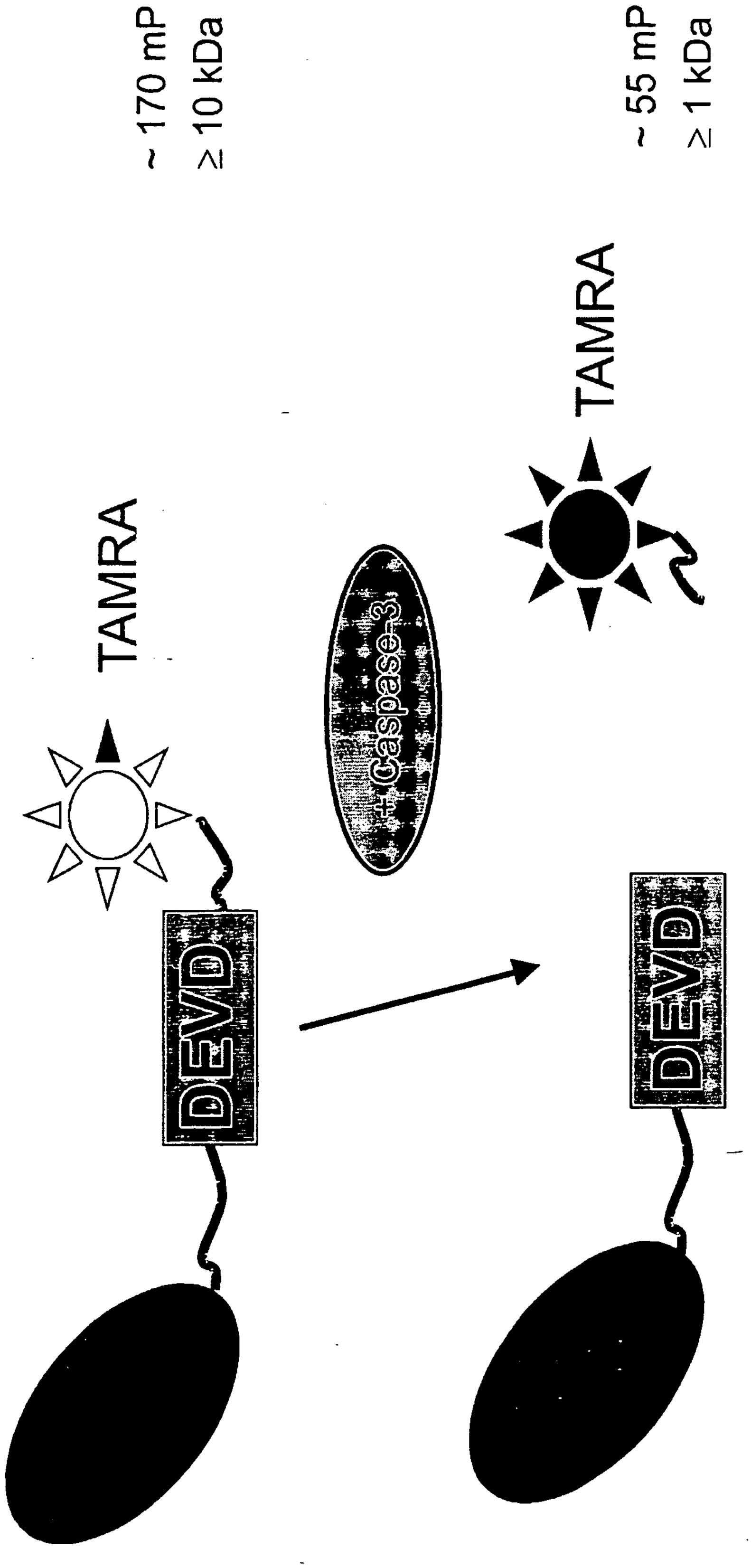


Fig.5

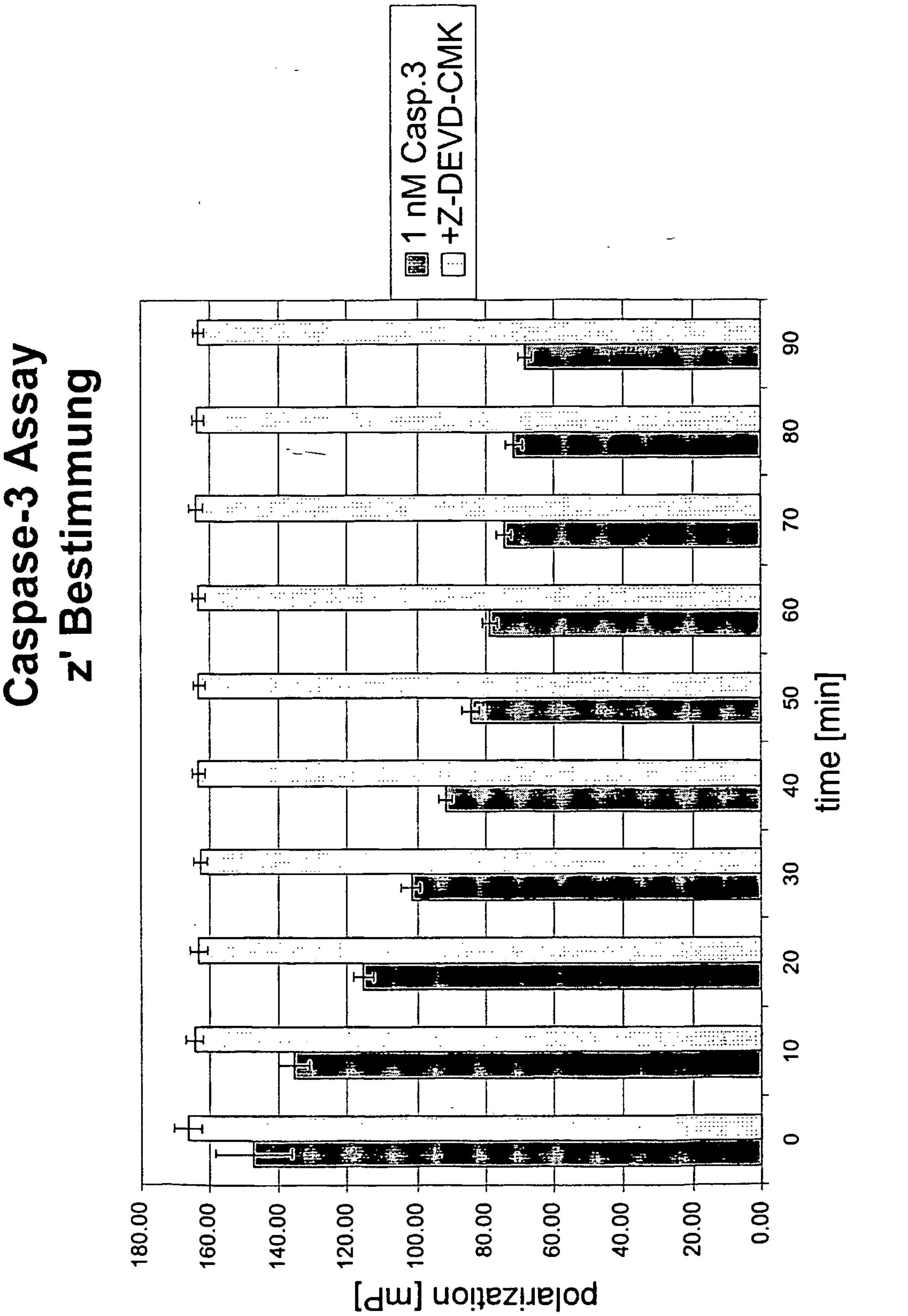


Z'≥ 0.83 nach 40 min

Fig.6

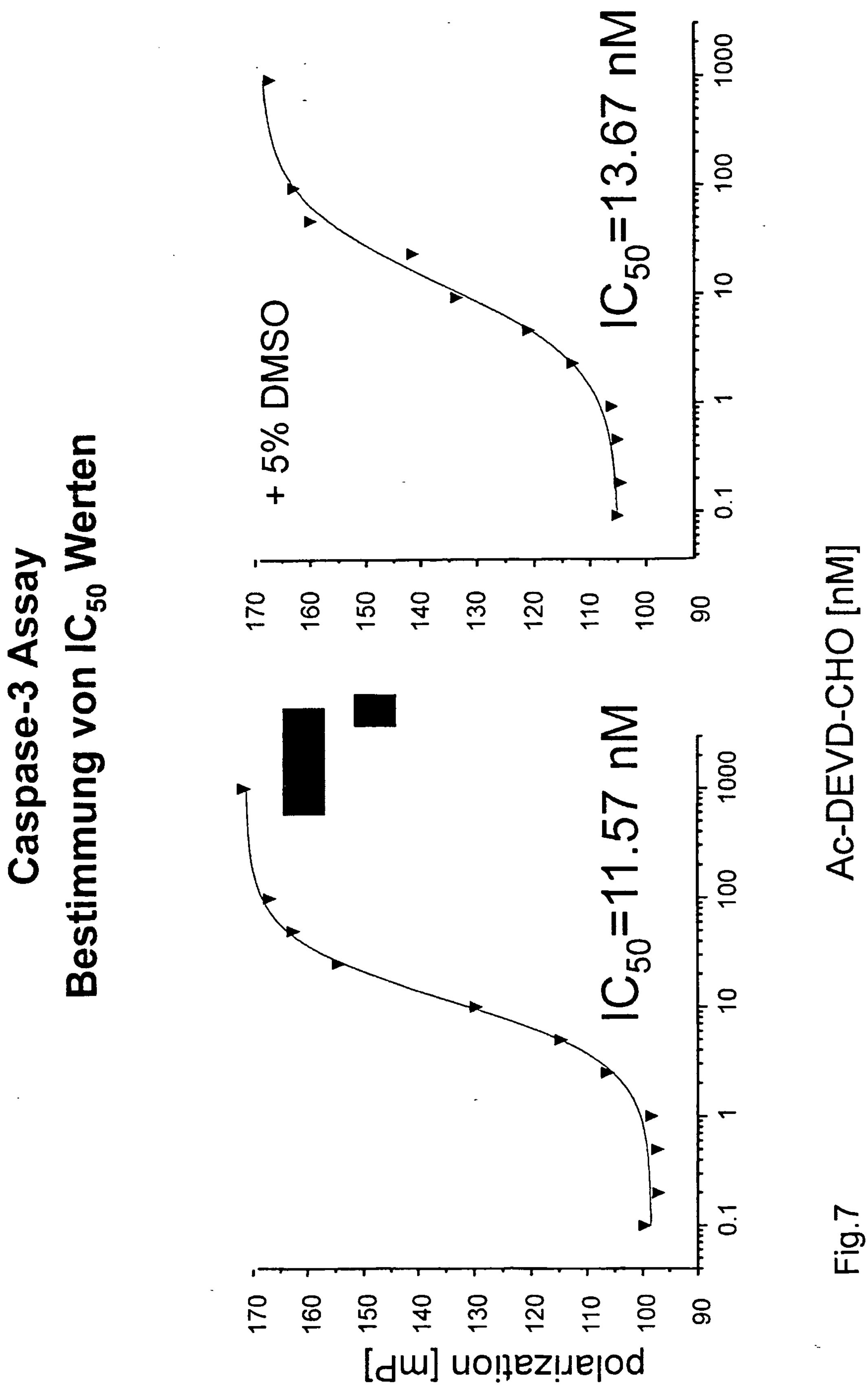
Caspase-3 Assay
Bestimmung von IC50 Werten
polarization [mP]
170
160
150
140
130
120
110
100
90
0.1
1
10
100
1000
$IC_{50}$=11.57 nM
[redacted]
+ 5% DMSO
$IC_{50}$=13.67 nM
Ac-DEVD-CHO [nM]

Fig.7

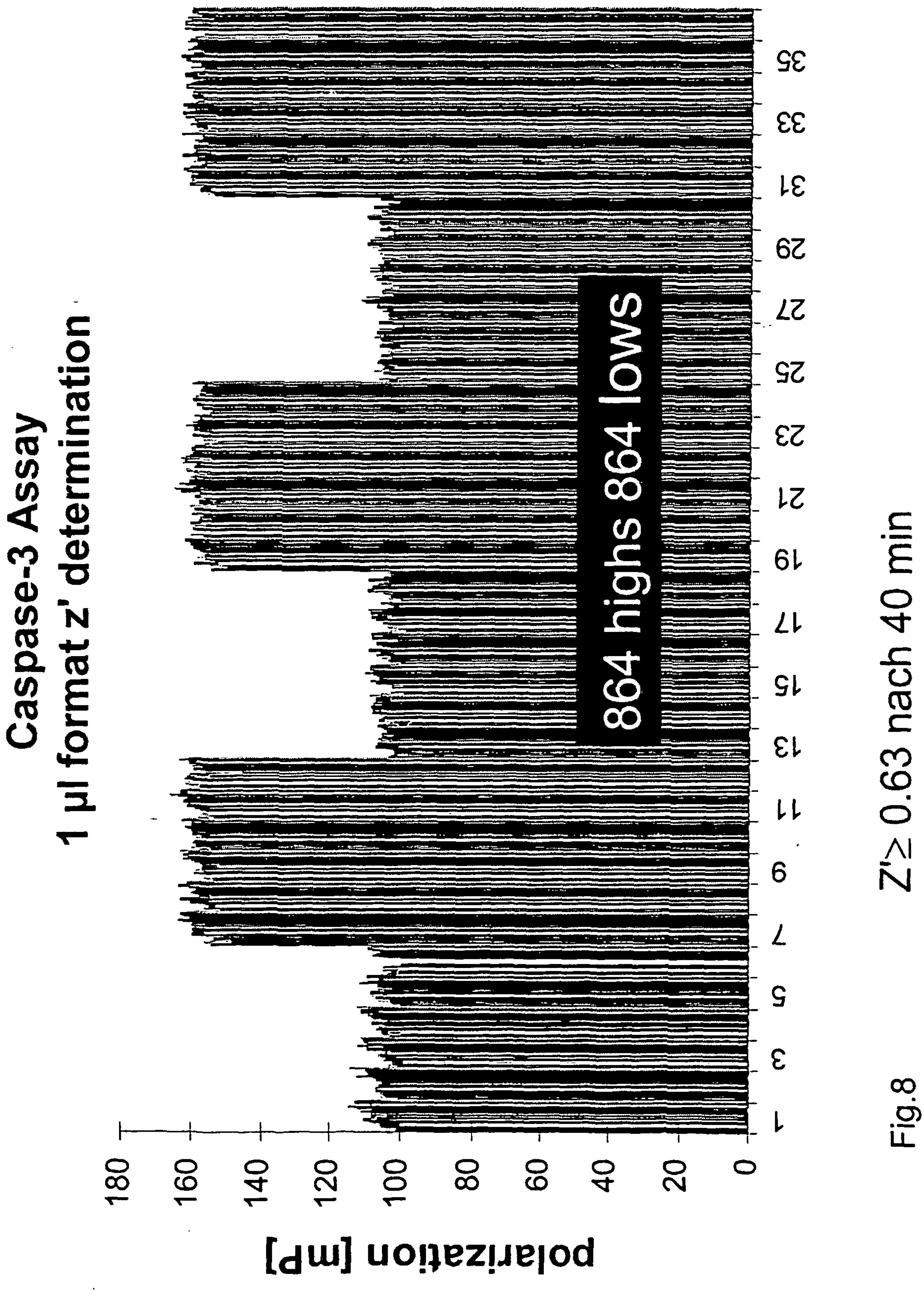
Caspase-3 Assay
1 µl format z' determination
864 highs 864 lows
polarization [mP]
0
20
40
60
80
100
120
140
160
180
1
3
5
7
9
11
13
15
17
19
21
23
25
27
29
31
33
35
Z'≥ 0.63 nach 40 min

Fig.8

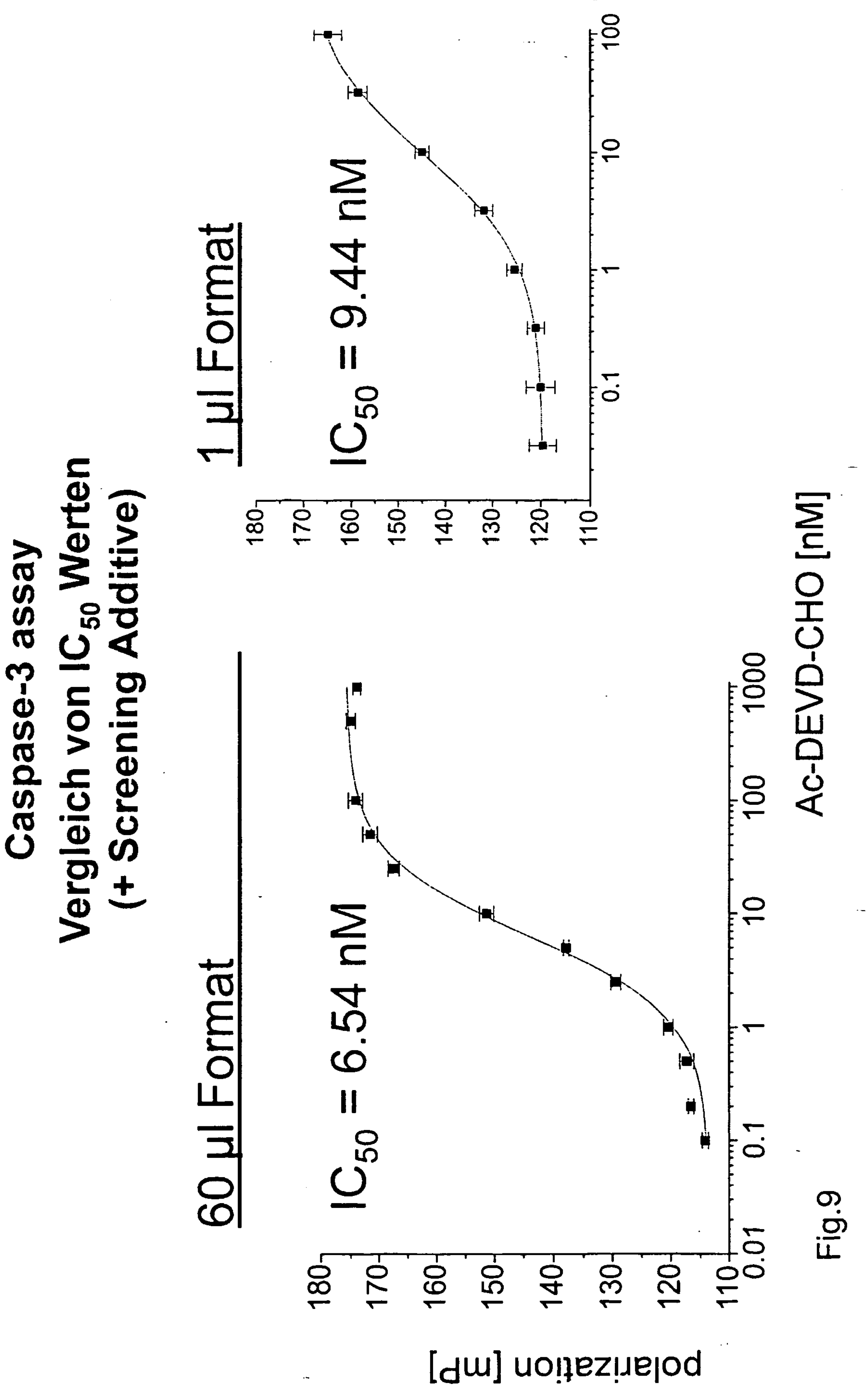
Caspase-3 assay
Vergleich von $IC_{50}$ Werten
(+ Screening Additive)
60 µl Format
$IC_{50}$ = 6.54 nM
1 µl Format
$IC_{50}$ = 9.44 nM
polarization [mP]
Ac-DEVD-CHO [nM]
180
170
160
150
140
130
120
110
0.01
0.1
1
10
100
1000

Fig.9

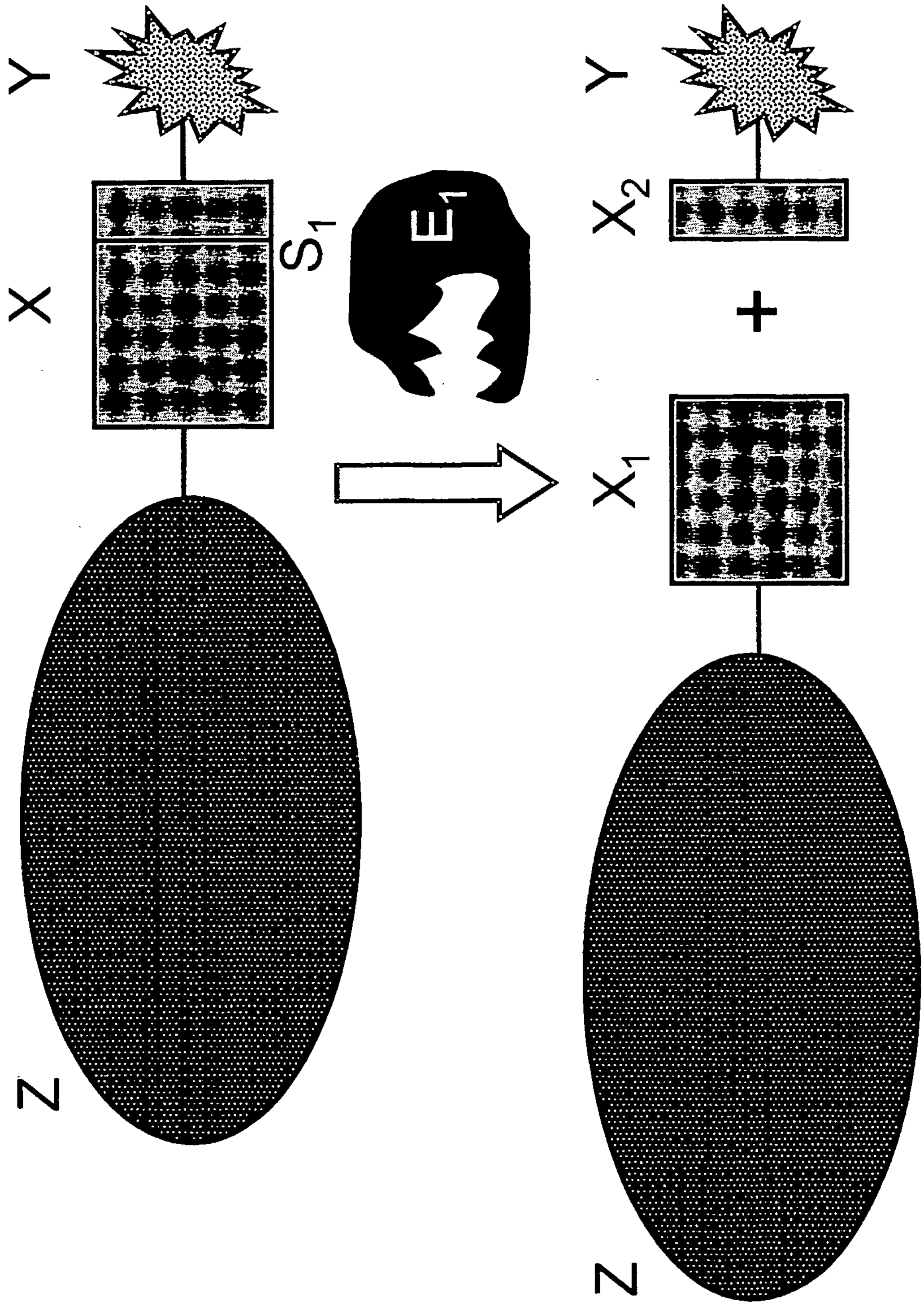


Fig. 10

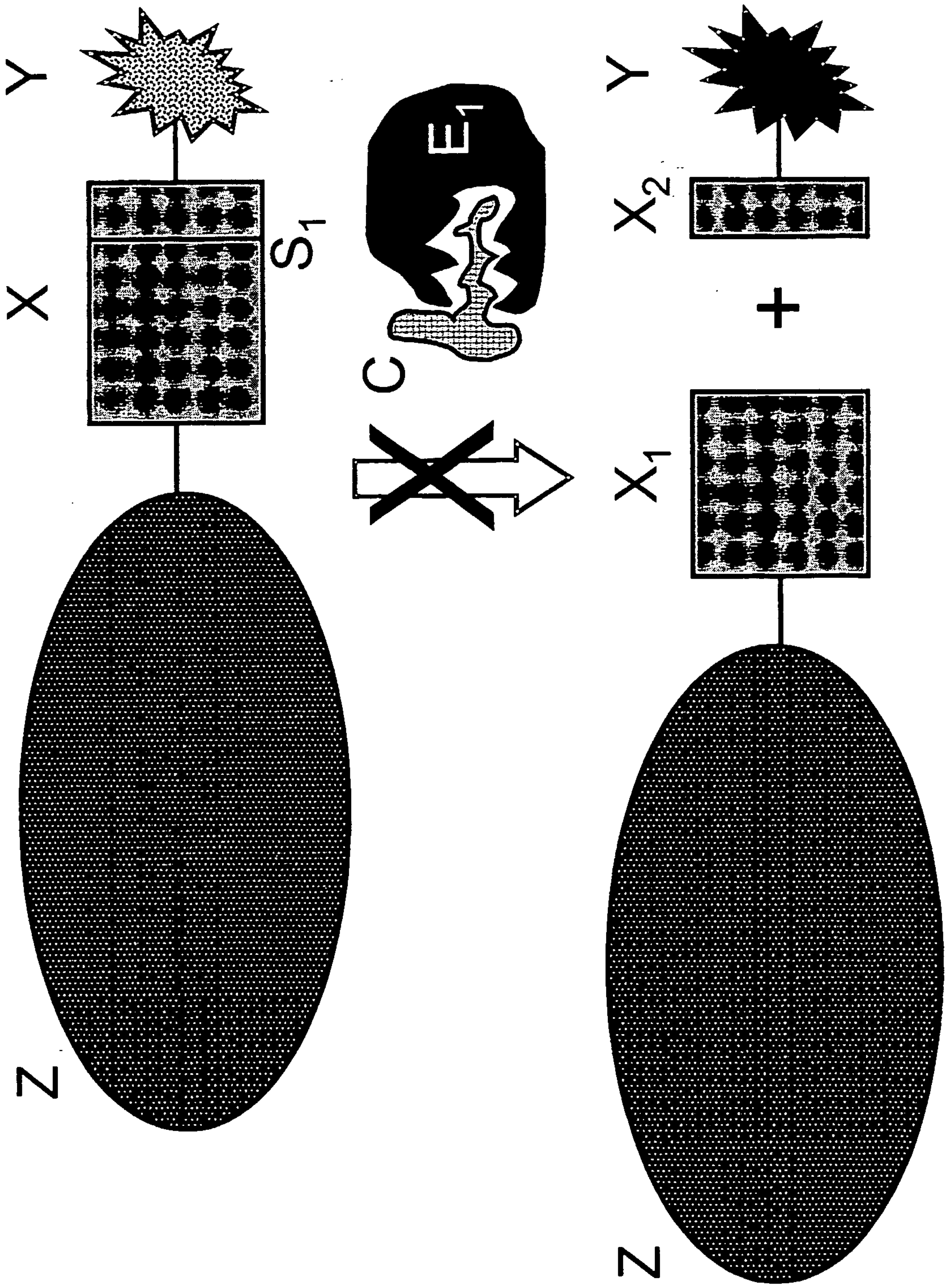


Fig.11

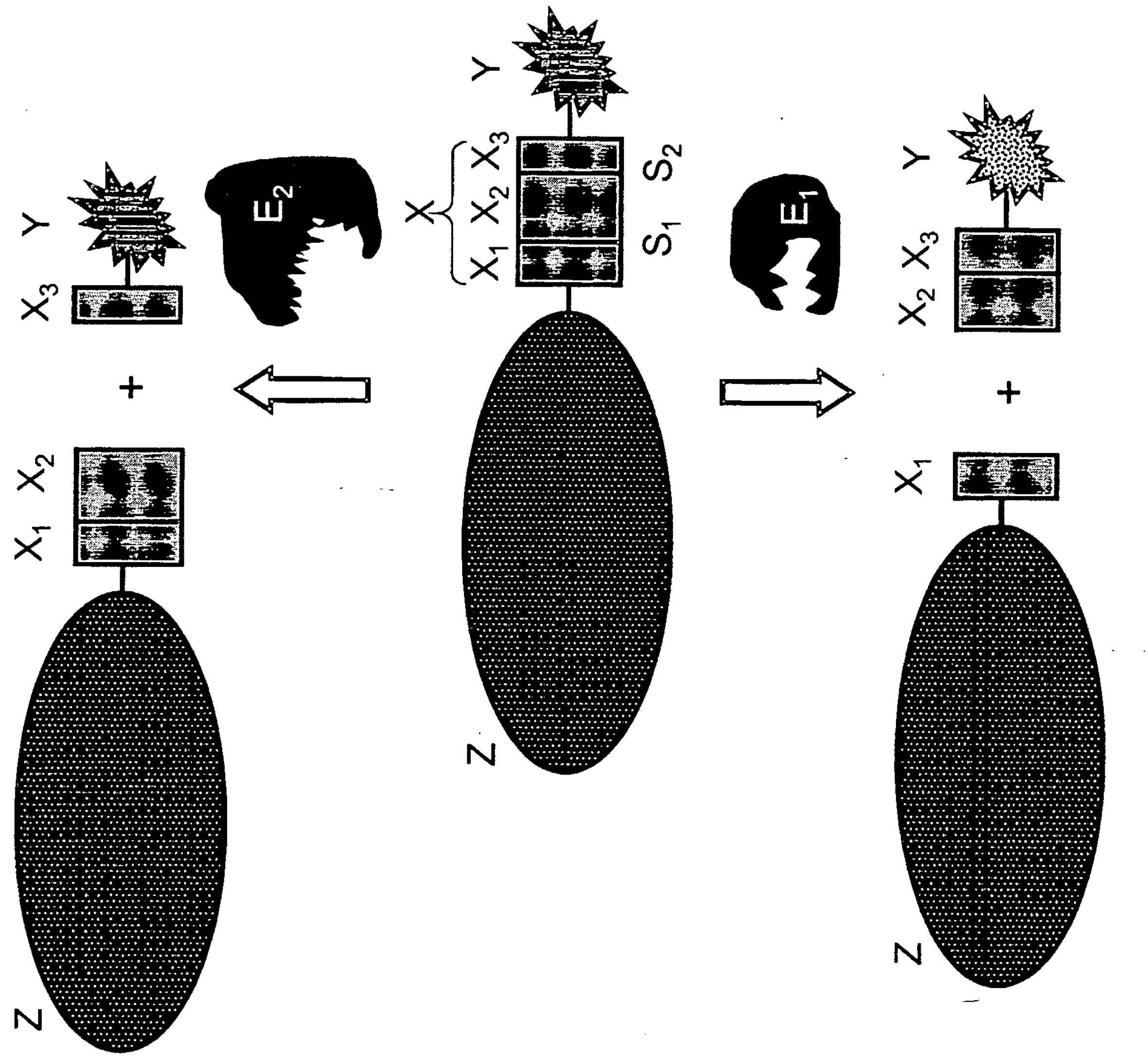


Fig.12

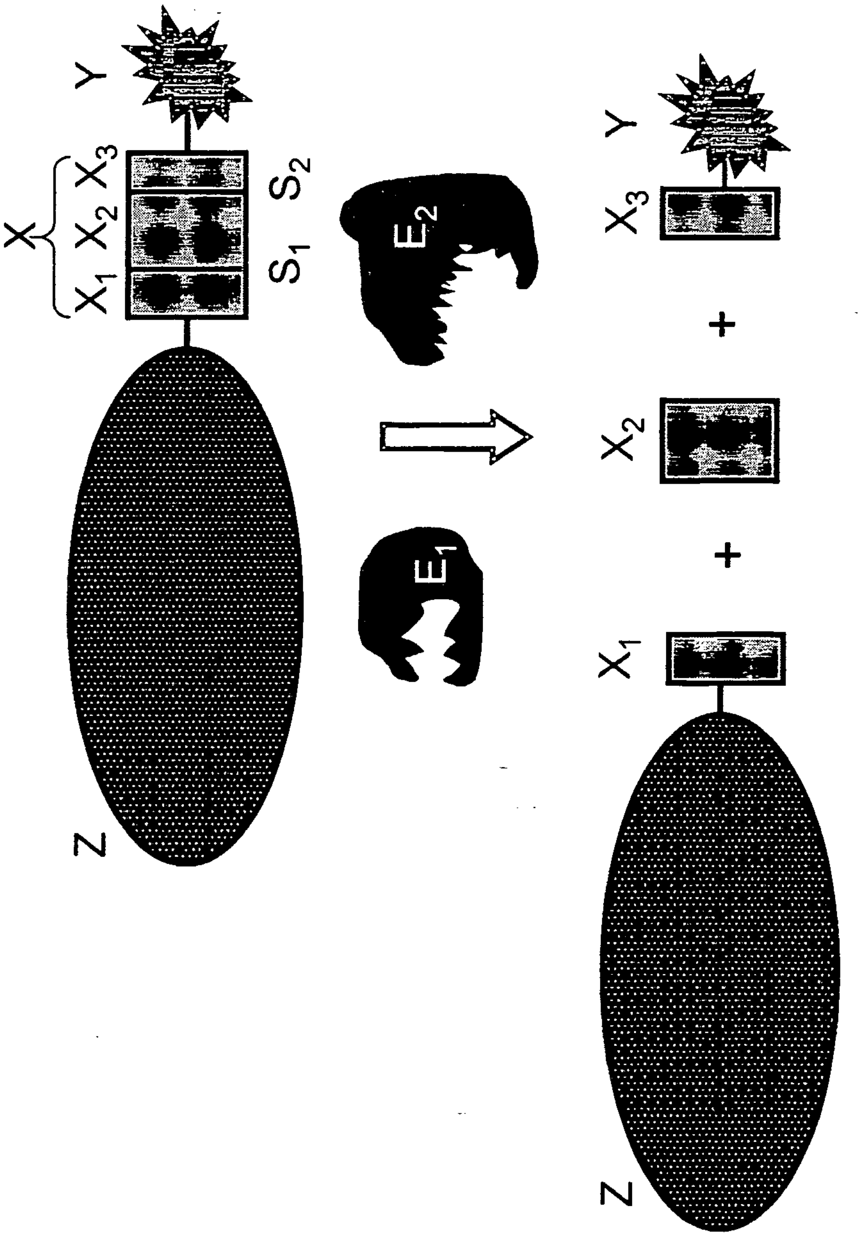


Fig.13

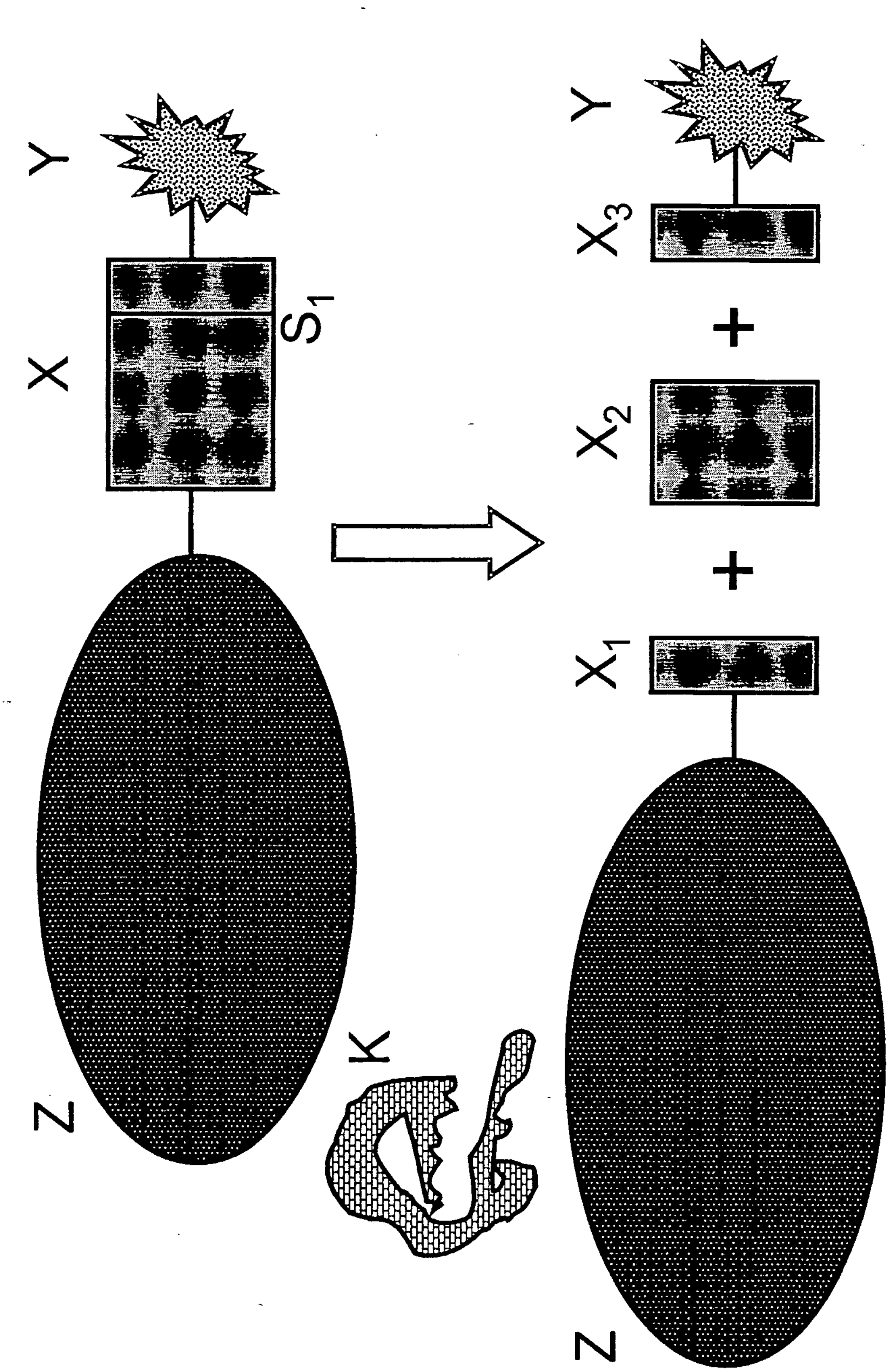


Fig.14

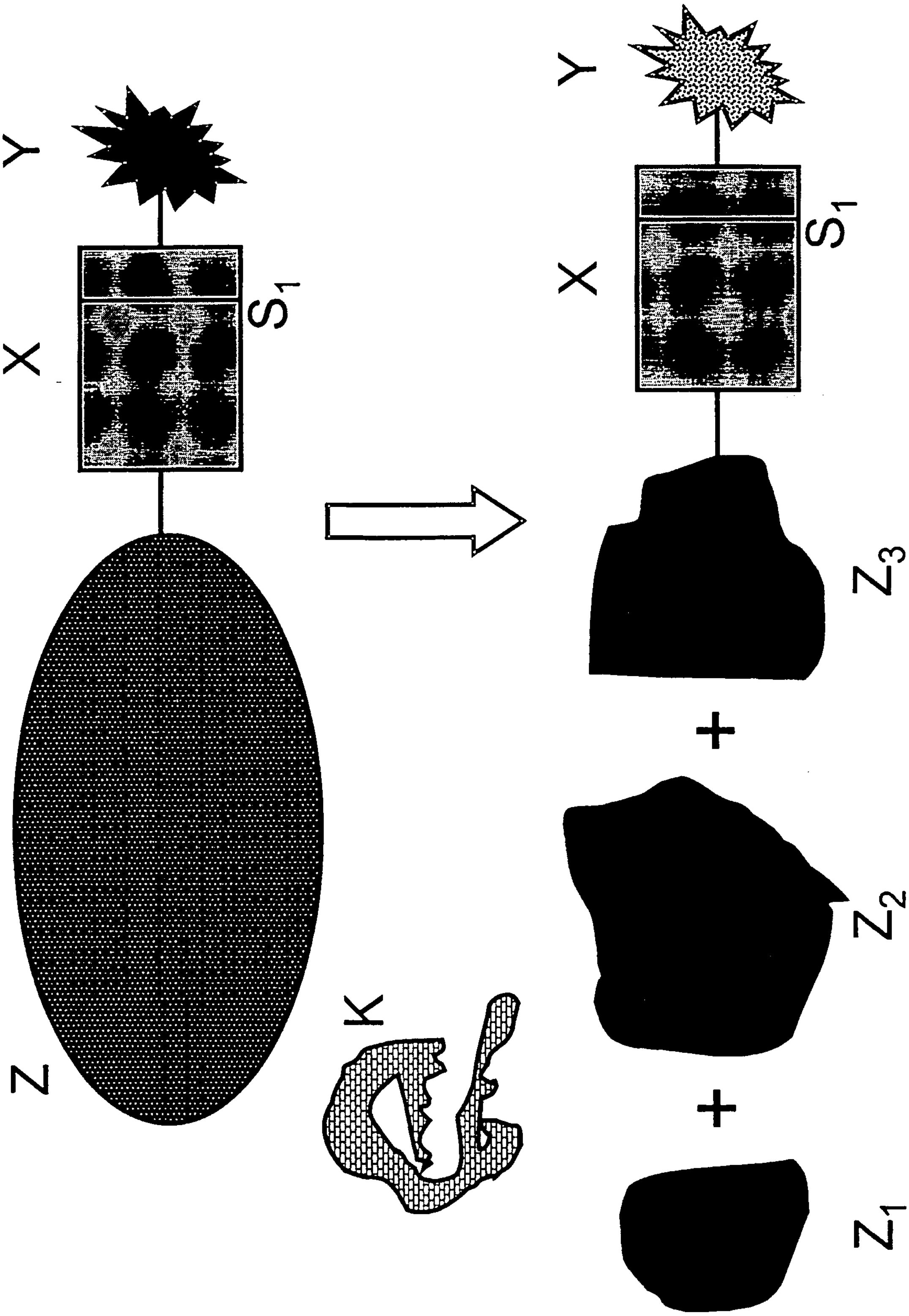


Fig.15

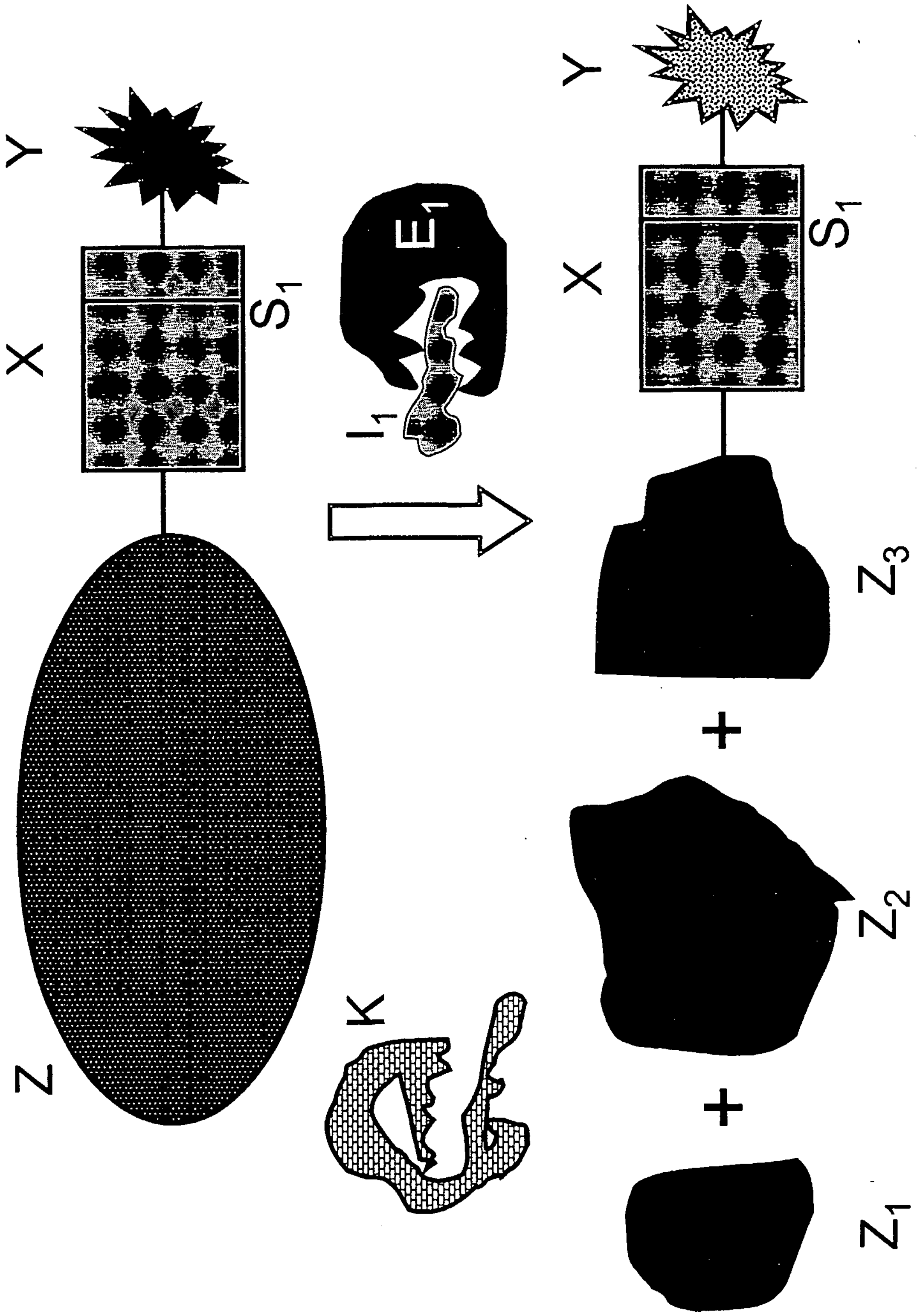


Fig.16

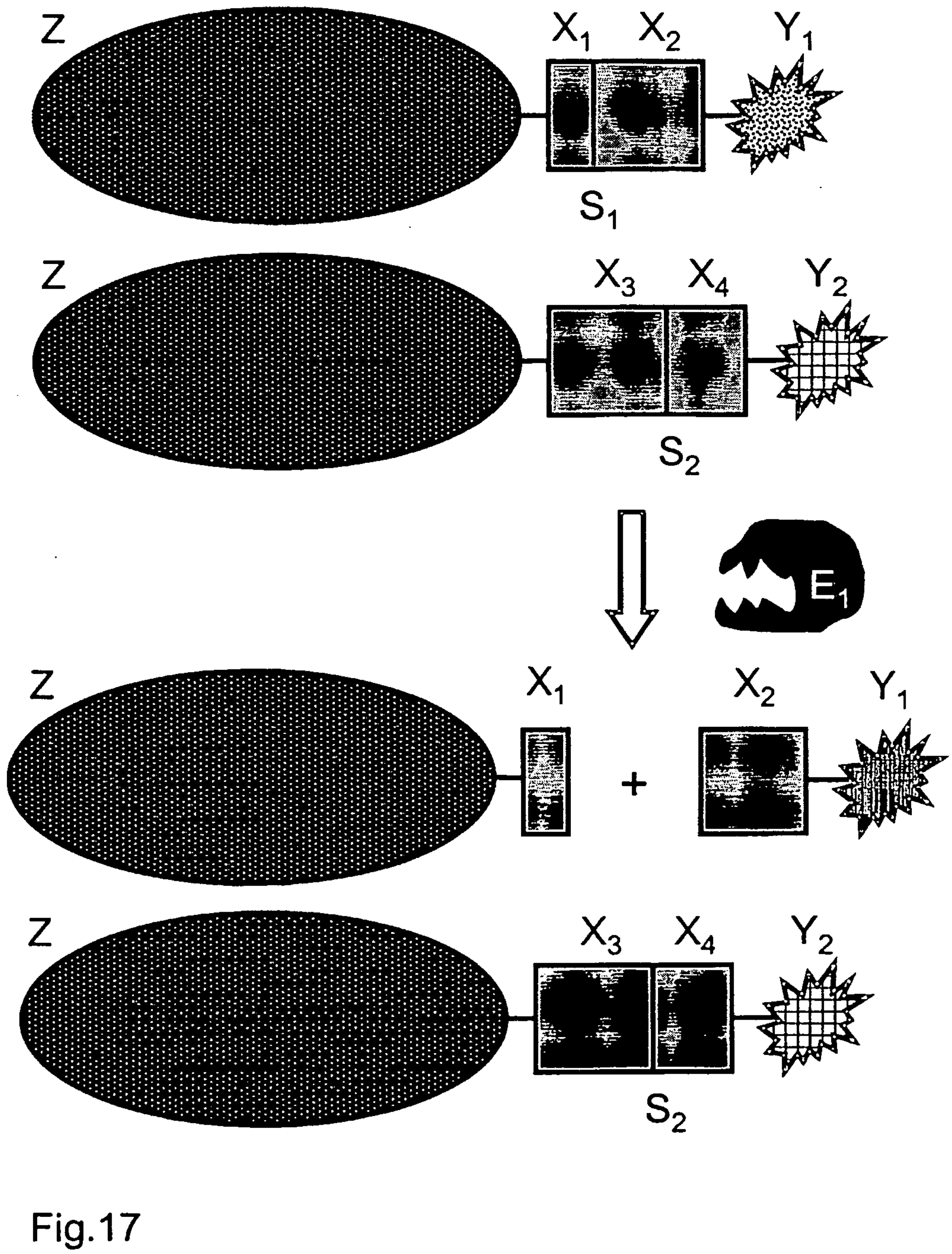


Fig.17

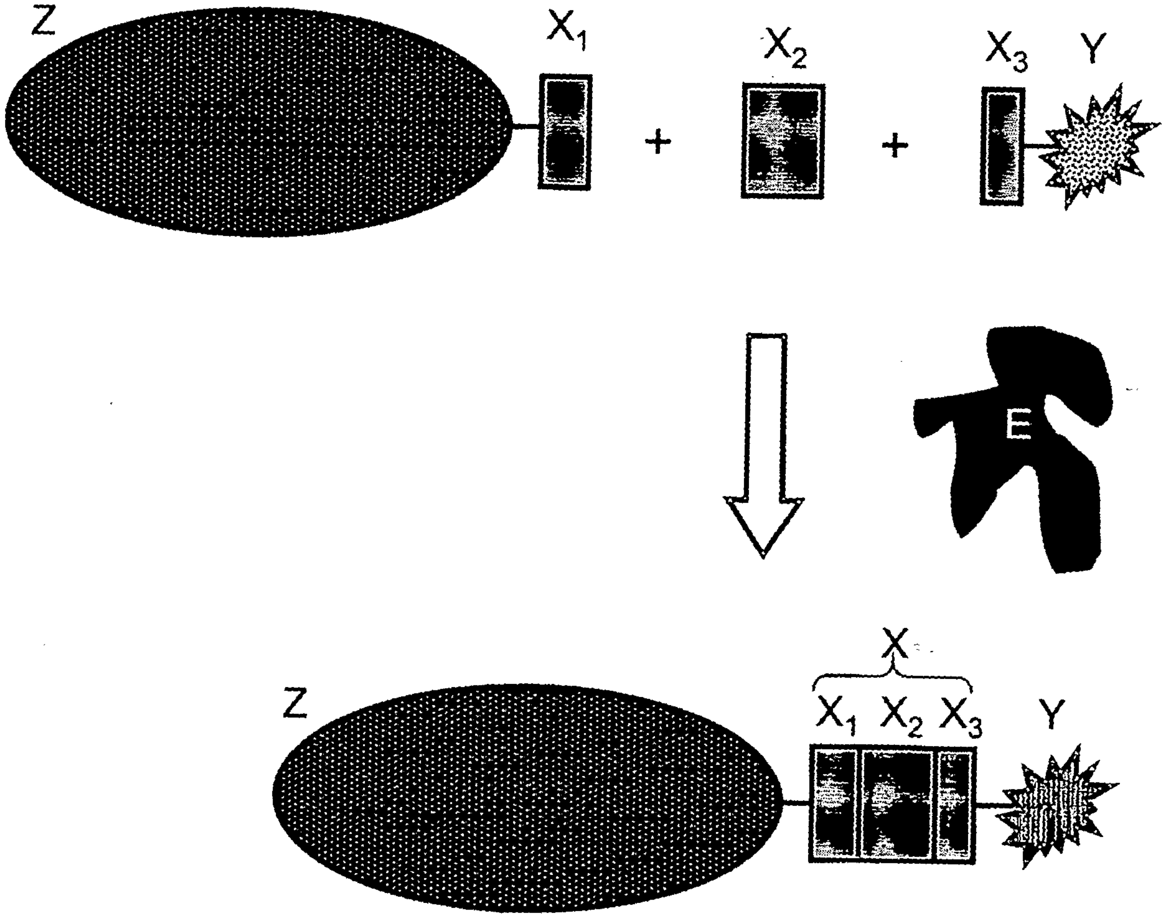


Fig.18

# Prozeß der Wirkstoffindung: Screening nach Inhibitoren für Caspase-3

- Anzahl der durchmusterten potentiellen Modulatoren: 193,146
- Modulatorenkonzentration: 17 µM
- Primärhitrate: 1.5%
- Anzahl identifizierter aktiver Modulatoren: 229
- mittlerer Z' Wert (Datenstatistik): 0.8
- Dose-response Analyse: 27 Modulatoren mit $IC_{50}$ Werten unter 50 µM identifiziert

Fig.19

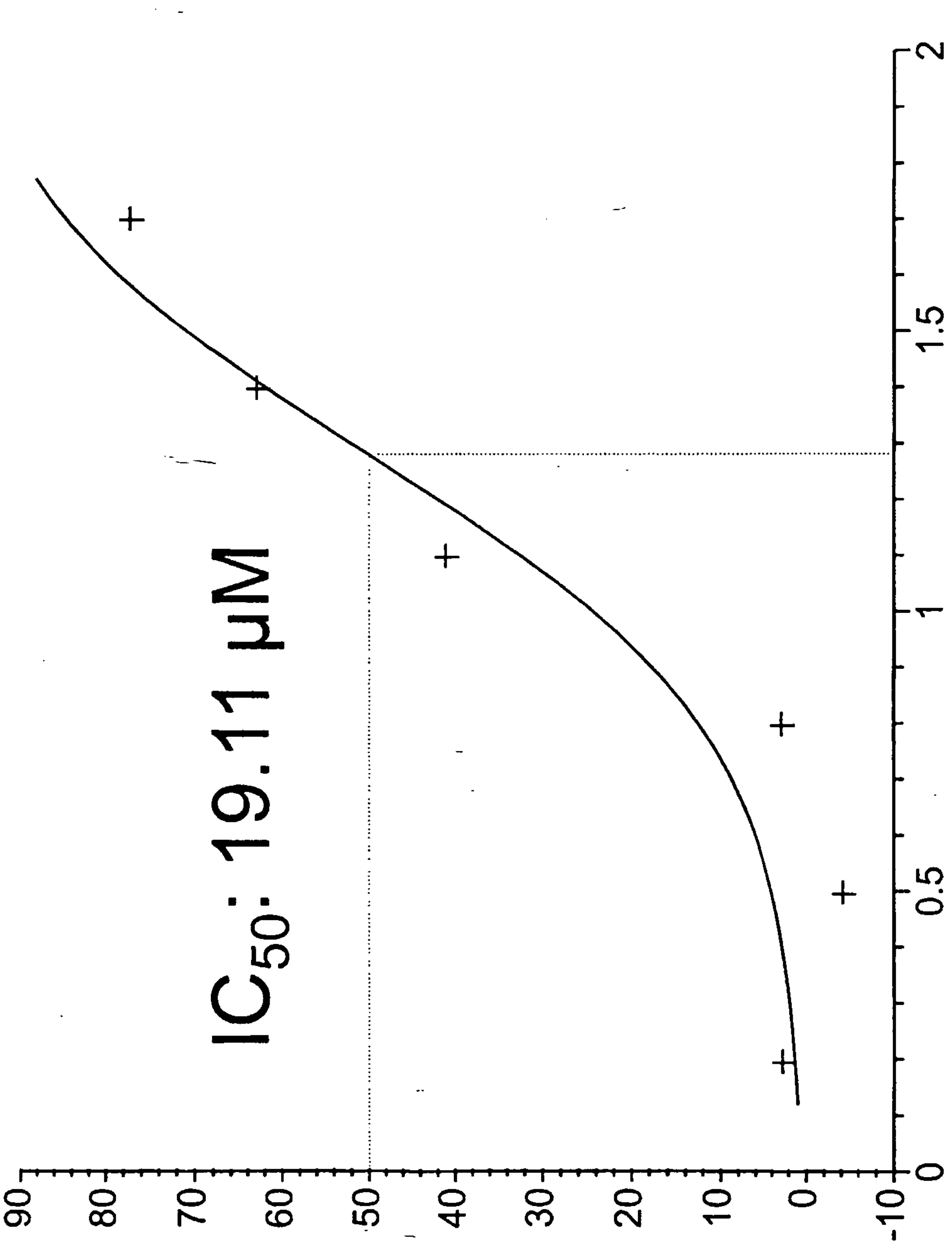


Fig.20

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4557862 A
- US 4640893 A
- WO 9918856 A
- WO 0072016 A


### In der Beschreibung aufgeführte Nicht-Patentliteratur


- **NJUS et al.** *Analytical Biochemistry,* 1974, vol. 61, 280-287
- **LEYTUS et al.** *Biochem. J.,* 1983, vol. 209, 299-307
- *Biochem. J.,* 1983, vol. 215, 253-260
- **HUG et al.** *Biochemistry,* 1999, vol. 38, 13906-13911
- **XIANG et al.** *Proc. Natl. Acad. Sci. USA,* 1996, vol. 93, 14559-14563
- **TALANIAN et al.** *The Journal of Biological Chemistry,* 1997, vol. 272, 9677-9682
- **PENNINGTON et al.** *Peptide Research,* 1994, vol. 7, 72-76
- *J. Phys. Chem. B,* 1998, vol. 102, 1820-1827
- *J. Phys. Chem. B,* 1998, vol. 102, 752-758
- *Biophysical Chemistry,* 1997, vol. 67, 167-176
- *Analytical Chemistry,* 1979, vol. 92, 222-227
- **BOLGER et al.** *BioTechniques,* 1994, vol. 17, 586-589
- **LEVINE et al.** *Analytical Biochemistry,* 1997, vol. 247, 83-88
- **K. K. SINGH ; T. RÜCKER ; A. HANNE ; R. PARWARESCH ; G. KRUPP.** *BioTechniques,* August 2000, vol. 29, 344-351
- **ZHANG, J.-H. et al.** *J. Biomol. Screen.,* 1999, vol. 4, 67-73